# EUROPEAN PATENT APPLICATION

(11) **EP 2 492 688 A1**
(43) Date of publication of application: **29.08.2012**
(21) Application number: 11382048.4
(22) Date of filing: 23.02.2011
(51) Int. Cl.: G01N 33/574, A61K 31/00

(54) **Molecular biomarkers for predicting response to antitumor treatment in lung cancer**

(71) Applicant: Pangaea Biotech, S.A., 08028 Barcelona (ES)
(72) Inventor: Tarón Roca, Miguel, 08028 Barcelona (ES); Rosell Costa, Rafael, 08028 Barcelona (ES)
(74) Representative: ABG Patentes, S.L.

(57) **Abstract**

The invention relates to a method for predicting the response to the treatment with an antitumor agent of a patient suffering lung cancer based on the expression levels in a sample of said patient of the AEG-1 gene or of the combination of the AEG-1 and BRCA1 genes wherein low expression levels of AEG-1 or low expression levels of AEG-1 and BRCA1 are indicative of a positive response of the patient to said antitumor agent.

## Description

### FIELD OF THE INVENTION

The invention relates to the field of pharmacogenomics and, more in particular, to the identification or markers capable of predicting the response of a patient suffering lung cancer to an antitumor therapy.

### BACKGROUND OF THE INVENTION

Non-small-cell lung cancer (NSCLC) accounts for approximately 80% of all lung cancers, with 1.2 million new cases worldwide each year. NSCLC resulted in more than one million deaths worldwide in 2001 and is the leading cause of cancer-related mortality in both men and women (31% and 25%, respectively). The prognosis of advanced NSCLC is dismal. A recent Eastern Cooperative Oncology Group trial of 1155 patients showed no differences among the chemotherapies used: cisplatin/paclitaxel, cisplatin/gemcitabine, cisplatin/docetaxel and carboplatin/paclitaxel. Overall median time to progression was 3.6 months, and median survival was 7.9 months.

At diagnosis, patients with NSCLC can be divided into three groups that reflect both the extent of the disease and the treatment approach:
■ The first group of patients has tumors that are surgically resectable (generally stage I, stage II, and selected stage III tumors). This group has the best prognosis.
■ The second group includes patients with either locally (T3-T4) and/or regionally (N2-N3) advanced lung cancer. Patients with unresectable or N2-N3 disease are treated with radiation therapy in combination with chemotherapy. Selected patients with T3 or N2 disease can be treated effectively with surgical resection and either preoperative or postoperative chemotherapy or chemoradiation therapy.
■ The final group includes patients with distant metastases (M1). This group can be treated with pallieative radiation therapy or chemotherapy.

The overall five-year survival of patients with NSCLC has remained at less than 15% for the past 20 years. Stage grouping of TNM subsets (T=primary tumor; N=regional lymph nodes; M=distant metastases) permits the identification of patient groups with similar prognosis and treatment options. Five-year survival is around 25% for pathologic stage IIB (T1-2N1M0, T3N0M0), 13% for stage IIIA (T3N1M0, T12-3N2M0), and a low 7% for stage IIIB (T4N0-1-2M0).

Multiple studies have attempted to identify prognostic determinants after surgery and have yielded conflicting evidence as to the prognostic importance of a variety of clinicopathologic factors. Factors that have been observed to correlate with adverse prognosis include the following:
- Presence of pulmonary symptoms.
- Large tumor size (>3 cm).
- Non-squamous histology.
- Metastases to multiple lymph nodes within a TNM-defined nodal station.
- Vascular invasión.
- Increased numbers of tumor blood vessels in the tumor specimen.

For the past several years, efforts have been focused on the development of targeted therapy direct against EGFR in non-small cell lung carcinoma (NSCLC). EGFR is present in the majority of NSCLCs. It is a member of the ErbB family of closely related receptors including EGFR (ErbB-1), Her2/neu (ErbB-2), Her3 (ErbB-3) and Her4 (ErbB-4). Activation of EGFR leads to receptor tyrosine kinase activation and a series of downstream signaling events that mediate increases in cellular proliferation, motility, adhesion, invasion, blocking of apoptosis and resistance to chemotherarapy. EGFR and its ligands, EGF and transforming growth factor alpha, are expressed in over 80% of NSCLC. Upon ligand binding, EGFR homodimerizes or forms heterodimers with other members of the ErbB family leading to receptor phosphorylation and activation of downstream signaling events. EGFR activation leads to the association with multiple signaling mediators such as She, Grb2, Src, JAKs, PLD, PLCGAMMA, and PI3K and subsequently to the activation of signaling transducers such as ERK1/2, FAK, JNK, STATs, and Akt. The importance of EGFR in tumorigenesis has prompted the development and commercialization of therapeutic agents that block its function.

The recent treatment success of gefitinib (Iressa) and erlotinib (Tarceva), two small molecule inhibitors of EGFR, in a fraction of patients with NSCLC has further solidified the premise that EGFR is a valid target. Several groups have independently identified frequent somatic mutations in the kinase domain of the EGFR gene in lung adenocarcinoma. These occur in 16% of lung adenocarcinoma specimens sequenced in the U.S. and 40% of those sequenced in Asia. The mutations are associated with sensitivity to both gefitinib and erlotinib, explaining in part the rare and dramatic clinical responses to treatment with these agents. Subsequent studies by multiple groups have now identified EGFR kinase domain mutations from many additional lung cancer patients. These mutations cluster in four groups, or regions; exon 19 deletions, exon 20 insertions, and point mutations at G719S and L858R. Thus far, the incidence of these kinase domain mutations is more common in adenocarcinomas than in lung cancers of other histological subtypes such as squamous cell carcinoma.

Recent emerging data also suggest that EGFR expression assessed by immunohistochemistry and the EGFR gene copy number might play an equally important role in identifying patients more likely to respond and have longer survival when treated with gefitinib or erlotinib.

However, nearly all patients who initially respond to erlotinib and gefitinib subsequently relapse. The importance of indentifying early in time the patients that are suffering or will suffer a relapse before the physical relapse symptoms appear (cough, pain or tumoral mass observed by PET/CT) is very high since it will allow the practitioners to choose an alternative therapy. An early diagnosis of the relapse will result in an increase of the survival rate of lung cancer patients.

WO2004071572 describes different marker genes, the expression levels of which are useful for determining the response to EGFR inhibitors in lung cancer patients. WO2009030605 describes that the expression levels of different ligandos of the VEGF receptor and, in particular, VEGF-1 and FLT-1, correlate with survival in ling cancer patients which have been treated with EGFR inhibitors. Moreover, K-ras mutations has been shown to correlate with lack of sensitivity to erlotinib or gefitinib (see Pao et al., PLos Med., 2005, 2: e17).

However, there is a need in the art for further prognosis markers suitable for predicting with increased sensitivity the clinical outcome of a patient suffering from NSCLC to an antitumor treatment.

### SUMMARY OF THE INVENTION

In a first aspect, the invention relates to a method for predicting the response of a patient suffering lung cancer to an antitumor composition which comprises
(i) determining in a sample isolated from said patient the expression levels of the AEG-1 gene and
(ii) comparing the expression levels of said AEG-1 gene obtained in step (i) with a reference sample
   wherein a decreased expression level of the AEG-1 gene with respect to a reference sample is indicative of a good response to the antitumor treatment or wherein an increased expression level of AEG-1 gene with respect to a reference sample is indicative of a bad response to the antitumor composition.

In a second aspect, the invention relates to An antitumor composition for use in the treatment of lung cancer wherein the patient to be treated shows low expression levels of the AEG-1 gene.

In a third aspect, the invention relates to a kit comprising
(i) reagents for detecting the expression levels of AEG-1 and
(ii) reagents for detecting the expression levels of BRCA1.

### BRIEF DESCRIPTION OF THE FIGURES

**Figure 1****:** Kaplan-Meier curves of progression-free survival in non-small-cell lung cancer patients with EGFR mutations according to AEG-1 mRNA levels.
**Figure 2****:** Kaplan-Meier curves of disease-free survival in non-small-cell lung cancer patients with EGFR mutations according to AEG-1 and BRCA1 mRNA levels.
**Figure 3****:** Kaplan-Meier curves of progression-free survival by median values in non-small-cell lung cancer patients under standard of care according to AEG-1 mRNA levels.
**Figure 4****:** Kaplan-Meier curves of progression-free survival by tertiles in non-small-cell lung cancer patients under standard of care according to AEG-1 mRNA levels.
Figure 5: Kaplan-Meier curves of progression-free survival in non-small-cell lung cancer patients under standard of care according to AEG-1 mRNA and BRCA1 expression.

### DETAILED DESCRIPTION OF THE INVENTION

### Method for determining the response of a lung cancer patient to an EGFR kinase inhibitor based on the expression levels of the AEG-1 gene

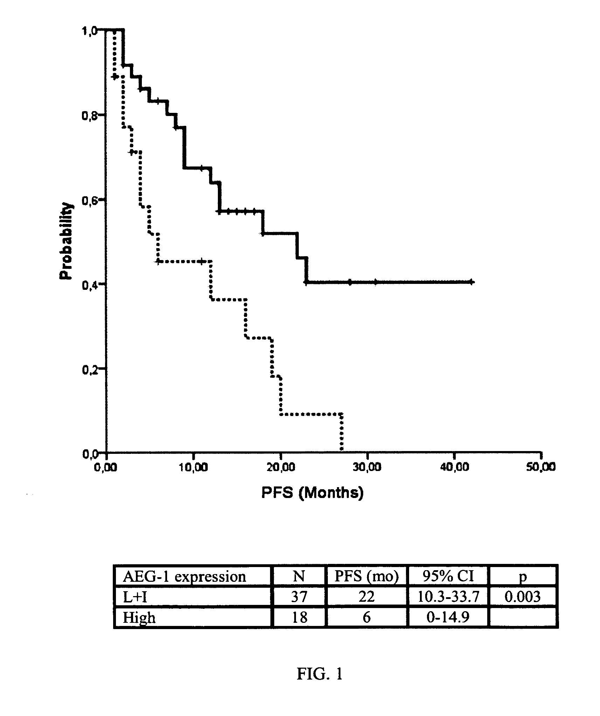

The authors of the present invention have observed that patients suffering lung cancer show an improved response to the therapy with an EGFR tyrosine kinase inhibitor when the expression levels of the either the AEG-1 gene measured in a sample from the patient are lower than those found in a reference sample. As shown in the example of the present invention, response rate was 67.6%, PFS was 16 months (m), median survival (MS) was 29 m. Median PFS was not reached in patients with low levels of AEG-1, while it was 18 months for patients with intermediate levels and 5 m for patients with high levels (P=0.002) (see Fig. 1).

This finding allows the prediction of the response to an EGFR tyrosine kinase inhibitor as well as to design personalized therapy for lung cancer patients based on the expression levels of AEG-1.

Thus, in a first aspect, the invention relates to a method (hereinafter first response determination method of the invention) for predicting the response of a patient suffering lung cancer to an antitumor treatment which comprises
(i) determining in a sample isolated from said patient the expression levels of the AEG-1 gene and
(ii) comparing the expression levels of said AEG-1 gene obtained in step (i) with a reference sample
   wherein a decreased expression level of the AEG-1 gene with respect to a reference sample is indicative of a good response to the antitumor treatment or
   wherein an increased expression level of AEG-1 gene with respect to a reference sample is indicative of a bad response to the antitumor treatment.

The term "predicting the response", as used herein refers to the determination of the likelihood that the patient will respond either favorably or unfavorably to a given therapy. Especially, the term "prediction", as used herein, relates to an individual assessment of any parameter that can be useful in determining the evolution of a patient. As will be understood by those skilled in the art, the prediction of the clinical response to the antitumor treatment, although preferred to be, need not be correct for 100% of the subjects to be diagnosed or evaluated. The term, however, requires that a statistically significant portion of subjects can be identified as having an increased probability of having a positive response. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably, 0.2, 0.1 or 0.05.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The term "clinical response", as used herein, refers to the response of the subject suffering from NSCLC to a therapy with a tyrosine kinase inhibitor. Standard criteria (Miller, et al. Cancer, 1981; 47:207-14) that can be used herewith to evaluate the response to chemotherapy include response, stabilization and progression. It can be a complete response (or complete remission) which is the disappearance of all detectable malignant disease or a partial response which is defined as approximately >50% decrease in the sum of products of the largest perpendicular diameters of one or more lesions (tumor lesions), no new lesions and no progression of any lession. Patients achieving complete or partial response were considered "responders", and all other patients were considered "non-responders".

The term "Stabilization", as used herein, is defined as a <50% decrease of a >25% increase in tumor size.

The term "Progression", as used herein, is defined as an increased in the size of tumor lesions by 25% or appearance of new lesions.

Any other parameter which is widely accepted for comparing the efficacy of alternative treatments can be used for determining a response to treatment and include, without limitation:
- disease-free progression which, as used herein, describes the proportion of patients in complete remission who have had no recurrence of disease during the time period under study,
- disease-free survival (DFS), as used herewith, is understood as the length of time after treatment for a disease during which a subject survives with no sign of the disease.
- objective response which, as used in the present invention, describes the proportion of treated people in whom a complete or partial response is observed.
- tumor control, which, as used in the present invention, relates to the proportion of treated people in whom complete response, partial response, minor response or stable disease ≥ 6 months is observed.
- progression free survival which, as used herein, is defined as the time from start of treatment to the first measurement of cancer growth.
- Time to progression (TTP), as used herein, relates to the time after a disease is treated until the disease starts to get worse. The term "progression" has been previously defined.

The response in individual patients may be characterized as a complete response, a partial response, stable disease, and progressive disease, as these terms are understood in the art. In certain embodiments, the response is a pathological complete response. A pathological complete response, e.g., as determined by a pathologist following examination of tissue removed at the time of surgery or biopsy, generally refers to an absence of histological evidence of invasive tumor cells in the surgical specimen. In particular, response may be determined by observing partial or total disappearance of one or more sings and symptoms associated with lung cancer such as difficulty in breathing, cough, shortness of breath, wheezing, chest pain and hemoptysis.

The term "lung cancer" is meant to refer to any cancer of the lung and includes non-small cell lung carcinomas and small cell lung carcinomas. In a preferred embodiment, the methods of the invention are applicable to a subject suffering from NSCLC. In a particular embodiment, the NSCLC is advanced NSCLC. In a still more preferred embodiment, the NSCLC is selected from squamous cell carcinoma of the lung, large cell carcinoma of the lung, and adenocarcinoma of the lung. Furthermore, the present method can also be applicable to a subject suffering from any stage of NSCLC (stages 0, IA, IB, IIa, IIb, IIIa, IIIb o IV).

The terms "antitumor treatment" and "antitumor agent", as used herein, refers to that chemical, physical or biological agent or compound with antiproliferative, antioncogenic and/or carcinostatic properties which can be used to inhibit tumor growth, proliferation and/or development. Examples of antitumor agents which can be used in the present invention are alkylating agents, antimetabolites, plant alkaloyds and terpenoids, topoisomerase inhibitors and the like.

In a preferred embodiment, the antitumor agent is platinum-based chemotherapeutic composition. The term "platinum-based chemotherapy", as used herein, refers to any chemotherapy using compounds which contain a platinum atom and which are capable of modifying the DNA inducing the activation of the DNA repair and subsequent cell death. Suitable platinum-based chemotherapeutic compounds include, without limitation, cisplatin, ELOXATIN (oxaliplatin), eptaplatin, lobaplatin, nedaplatin, PARAPLATIN (carboplatin), carboplatin, cisplatin, , iproplatin, tetraplatin, lobaplatin, DCP, PLD-147, JM1 18, JM216, JM335, and satraplatin. The term "platinum-based chemotherapy" also includes compositions of two or more chemotherapeutic agents wherein at least one of the components is a platinum-based compound, such as cisplatin-gemcitabine, carboplatin-gemcitabine, cisplatin-gemcitabine-vinorelbine, cisplatin-vinorelbine, cisplatin-etoposide, cisplatin-etoposide-vincristine, cisplatin-paclitaxel, cisplatin-docetaxel, carboplatin-docetaxel and the like.

In a preferred embodiment, the antitumor treatment is a treatment using an EGFR inhibitor.

The term "EGFR inhibitor", as used herein, refers to any therapeutic regime which includes one or more compounds capable of inhibiting the activity of EGFR.

The terms "EGFR", "ErbB1" and "epidermal growth factor receptor" and are used interchangeably herein and refer to a tyrosine kinase which regulate signaling pathways and growth and survival of cells and which shows affinity for the EGF molecule. The ErbB family of receptors consists of four closely related subtypes: ErbB1 (epidermal growth factor receptor [EGFR]), ErbB2 (HER2/neu), ErbB3 (HER3), and ErbB4 (HER4) and variants thereof (e.g. a deletion mutant EGFR as in Humphrey et al. (Proc. Natl. Acad. Sci. USA, 1990, 87:4207-4211). In a preferred embodiment, the EGFR is human.

In a preferred embodiment, the EGFR inhibitor is a EGFR tyrosine kinase inhibitor. The expression "EGFR tyrosine kinase inhibitor", as used herein, relates to a chemical substance inhibiting "tyrosine kinase" which transfers a γ-phosphate group of ATP to a hydroxy group of a specific tyrosine in protein catalised by the tyrosine kinase domain of the receptor for epidermal growth factor (EGFR). Tyrosine kinase activity is measured by detecting phosphorylation of a protein. EGFR tyrosine kinase inhibitors are known in the art. For example, a tyrosine kinase inhibitor is identified by detecting a decrease the tyrosine mediated transfer phosphate from ATP to protein tyrosine residues.

The type of EGFR tyrosine kinase inhibitor therapy for use according to the method of the present invention is not particularly limiting and may include, without limitation, a dual EGFR inhibitor, a dual EGFR tyrosine kinase inhibitor or a EGFR tyrosine kinase inhibitor specific for EGFR carrying a resistance mutation.

The term "dual EGFR inhibitor", as used herein, refers to a composition which is capable of simultaneously inhibiting the tyrosine kinase activity of the intracellular domain of EGFR as well as its activation by the binding of the ligand to the extracellular domain. Illustrative and non-limitaative example of such an inhibitor is, e.g. the composition comprising cetuximab (C225) as inhibitor of the extracellular domain and erlotinib (E) as inhibitor of the tyrosine kinase activity of the intracellular domain.

The term "dual EGFR tyrosine kinase inhibitor", as used herein, refers to a compound which is capable of simultaneously inhibiting EGFR and HER2 activity. Examples of such compounds include the EGFR and HER2 inhibitor CI-1033 (formerly known as PD183805; Pfizer); the EGFR and HER2 inhibitor GW-2016 (also known as GW-572016 or lapatinib ditosylate; GSK); the EGFR and JAK 2/3 inhibitor AG490 (a tyrphostin); the EGFR and HER2 inhibitor ARRY-334543 (Array BioPharma); BIBW-2992, an irreversible dual EGFR/HER2 kinase inhibitor (Boehringer Ingelheim Corp.)

In a preferred embodiment, the EGFR inhibitor-based chemotherapy is an inhibitor of the EGFR tyrosine kinase. The tyrosine kinase inhibitor is for example an erbB tyrosine kinase inhibitor. Alternatively the tyrosine kinase inhibitor is an EGFR tyrosine kinase inhibitor. The tyrosine kinase inhibitor is a reversible tyrosine kinase inhibitor. Alternatively the tyrosine kinase inhibitor is an irreversible tyrosine kinase inhibitor. Reversible tyrosine kinase inhibitors include for example, HKI-272, BIBW2992, EKB-569 or CL-387,785 or mimetics or derivatives thereof. Other tyrosine kinase inhibitors include those described in U.S. Pat. Nos. 6,384,051, 6,288,082 and US Application No. 20050059678, each of which is hereby incorporated by reference in their entireties.

EGFR tyrosine kinase inhibitors include, for example quinazoline EGFR kinase inhibitors, pyrido- pyrimidine EGFR kinase inhibitors, pyrimido-pyrimidine EGFR kinase inhibitors, pyrrolo- pyrimidine EGFR kinase inhibitors, pyrazolo-pyrimidine EGFR kinase inhibitors, phenylamino- pyrimidine EGFR kinase inhibitors, oxindole EGFR kinase inhibitors, indolocarbazole EGFR kinase inhibitors, phthalazine EGFR kinase inhibitors, isoflavone EGFR kinase inhibitors, quinalone EGFR kinase inhibitors, and tyrphostin EGFR kinase inhibitors, such as those described in the following patent publications, and all pharmaceutically acceptable salts and solvates of said EGFR kinase inhibitors: International Patent Publication Nos. WO 96/33980, WO 96/30347, WO 97/30034, WO 97/30044, WO 97/38994, WO 97/49688, WO 98/02434, WO 97/38983, WO 95/19774, WO 95/19970, WO 97/13771, WO 98/02437, WO 98/02438, WO 97/32881, WO 98/33798, WO 97/32880, WO 97/3288, WO 97/02266, WO 97/27199, WO 98/07726, WO 97/34895, WO 96/31510, WO 98/14449, WO 98/14450, WO 98/14451, WO 95/09847, WO 97/19065, WO 98/17662, WO 99/35146, WO 99/35132, WO 99/07701, and WO 92/20642; European Patent Application Nos. EP 520722, EP 566226, EP 787772, EP 837063, and EP 682027; U.S. Pat. Nos. 5,747,498, 5,789,427, 5,650,415, and 5,656,643; and German Patent Application No. DE 19629652. Additional non-limiting examples of low molecular weight EGFR kinase inhibitors include any of the EGFR tyrosine kinase inhibitors described in Traxler, P., 1998, Exp. Opin. Ther. Patents 8(12): 1599-1625.

Specific preferred examples of low molecular weight EGFR tyrosine kinase inhibitors that can be used according to the present invention include [6,7-bis(2-methoxyethoxy)-4-quinazolin-4-yl]- (3-ethynylphenyl)amine (also known as OSI-774, erlotinib, or TARCEVA.RTM. (erlotinib HCl); OSI Pharmaceuticals/Genentech/Roche) (U.S. Pat. No. 5,747,498; International Patent Publication No. WO 01/34574, and Moyer, J. D. et al. (1997) Cancer Res. 57:4838-4848); CI- 1033 (formerly known as PD183805; Pfizer) (Sherwood et al., 1999, Proc. Am. Assoc. Cancer Res. 40:723); PD-158780 (Pfizer); AG-1478 (University of California); CGP-59326 (Novartis); PKI-166 (Novartis); EKB-569 (Wyeth); GW-2016 (also known as GW-572016 or lapatinib ditosylate; GSK); and gefitinib (also known as ZD1839 or IRESSA.TM.; Astrazeneca) (Woodburn et al., 1997, Proc. Am. Assoc. Cancer Res. 38:633). A particularly preferred low molecular weight EGFR kinase inhibitor that can be used according to the present invention is [6,7-bis(2-methoxyethoxy)-4-quinazolin-4-yl]-(3-ethynylphenyl) amine (i.e. erlotinib), its hydrochloride salt (i.e. erlotinib HCl, TARCEVA.RTM.), or other salt forms (e.g. erlotinib mesylate).

EGFR tyrosine kinase inhibitors also include, for example multi-kinase inhibitors that have activity on EGFR kinase, i.e. inhibitors that inhibit EGFR kinase and one or more additional kinases. Examples of such compounds include the EGFR and HER2 inhibitor CI- 1033 (formerly known as PD 183805; Pfizer); the EGFR and HER2 inhibitor GW-2016 (also known as GW- 572016 or lapatinib ditosylate; GSK); the EGFR and JAK 2/3 inhibitor AG490 (a tyrphostin); the EGFR and HER2 inhibitor ARRY-334543 (Array BioPharma); BIBW-2992, an irreversible dual EGFR/HER2 kinase inhibitor (Boehringer Ingelheim Corp.); the EGFR and HER2 inhibitor EKB-569 (Wyeth); the VEGF-R2 and EGFR inhibitor ZD6474 (also known as ZACTIMA.TM.;AstraZeneca Pharmaceuticals), and the EGFR and HER2 inhibitor BMS-599626 (Bristol-Myers Squibb).

Antibody-based tyrosine EGFR kinase inhibitors include any anti-EGFR antibody or antibody fragment that can partially or completely block EGFR activation by its natural ligand. Non- limiting examples of antibody-based EGFR kinase inhibitors include those described in Modjtahedi, H., et al., 1993, Br. J. Cancer 67:247-253; Teramoto, T., et al., 1996, Cancer 77:639-645; Goldstein et al., 1995, Clin. Cancer Res. 1:1311-1318; Huang, S. M., et al., 1999, Cancer Res. 15:59(8):1935-40; and Yang, X., et al., 1999, Cancer Res. 59:1236-1243. Thus, the EGFR kinase inhibitor can be the monoclonal antibody Mab E7.6.3 (Yang, X. D. et al. (1999) Cancer Res. 59: 1236-43), or Mab C225 (ATCC Accession No. HB-8508), or an antibody or antibody fragment having the binding specificity thereof. Suitable monoclonal antibody EGFR kinase inhibitors include, but are not limited to, IMC-C225 (also known as cetuximab or ERBITUX.TM.; Imclone Systems), ABX-EGF (Abgenix), EMD 72000 (Merck KgaA, Darmstadt), RH3 (York Medical Bioscience Inc.), and MDX-447 (Medarex/Merck KgaA).

In another embodiment, an antisense strategy may be used to interfere with the kinase activity of a variant EGFR. This approach may, for instance, utilize antisense nucleic acids or ribozymes that block translation of a specific mRNA, either by masking that mRNA with an antisense nucleic acid or cleaving it with a ribozyme. For a general discussion of antisense technology, see, e.g., Antisense DNA and RNA, (Cold Spring Harbor Laboratory, D. Melton, ed., 1988).

Reversible short inhibition of variant EGFR gene transcription may also be useful. Such inhibition can be achieved by use of siRNAs. RNA interference (RNAi) technology prevents the expression of gene∼ by using small RNA molecules such as small interfering RNAs (siRNAs). This technology in turn takes advantage of the fact that RNAi is a natural biological mechanism for silencing genes in most cells of many living organisms, from plants to insects to mammals (McManus et aI., Nature Reviews Genetics, 2002, 3(10) p. 737). RNAi prevents a gene from producing a functional protein by ensuring that the molecule intermediate, the messenger RNA copy of the gene is destroyed. siRNAs can be used in a naked form and incorporated in a vector, as described below. One can further make use of aptamers to specifically inhibit variant EGFR gene transcription, see, for example, U.S. Patent 6,699,843. Aptamers useful in the present invention may be identified using the SELEX process. The methods of SELEX have been described in, for example, U. S. Patent Nos. 5,707,796, 5,763,177, 6,011,577, 5,580,737, 5,567,588, and 5,660,985.

An "antisense nucleic acid" or "antisense oligonucleotide" is a single stranded nucleic acid molecule, which, on hybridizing under cytoplasmic conditions with complementary bases in a RNA or DNA molecule, inhibits the latter's role. If the RNA is a messenger RNA transcript, the antisense nucleic acid is a counter-transcript or mRNA-interfering complementary nucleic acid. As presently used, "antisense" broadly includes RNA-RNA interactions, RNA- DNA interactions, ribozymes, RNAi, aptamers and Rnase-H mediated arrest.

Ribozymes are RNA molecules possessing the ability to specifically cleave other single stranded RNA molecules in a manner somewhat analogous to DNA restriction endonucleases. Ribozymes were discovered from the observation that certain mRNAs have the ability to excise their own introns. By modifying the nucleotide sequence of these ribozymes, researchers have been able to engineer molecules that recognize specific nucleotide sequences in an RNA molecule and cleave it (Cech, 1989, Science 245(4915) p. 276). Because they are sequence-specific, only mRNAs with particular sequences are inactivated.

Antisense nucleic acid molecules can be encoded by a recombinant gene for expression in a cell (e.g., U.S. patent No 5,814,500; U.S. 5,811,234), or alternatively they can be prepared synthetically (e.g., u.s. patent No 5,780,607).

siRNAs have been described in Brummelkamp et al., Science 296; 550-553,2002, Jaque et al., Nature 418; 435-438, 2002, Elbashir S. M. et al. (2001) Nature, 411: 494-498, McCaffrey et al. (2002), Nature, 418: 38-39; Xia H. et al. (2002), Nat. Biotech. 20: 1006-1010, Novina et al. (2002), Nat. Med. 8: 681-686, and U.S. Application No. 20030198627.

An important advantage of such a therapeutic strategy relative to the use of drugs such as gefitinib, which inhibit both the mutated receptor and the normal receptor, is that siRNA directed specifically against the mutated EGFR should not inhibit the wildtype EGFR. This is significant because it is generally believed that the "side effects" of gefitinib treatment, which include diarrhea and dermatitis, are a consequence of inhibition of EGFR in normal tissues that require its function.

In another embodiment, the compounds are antisense molecules specific for human sequences coding for an EGFR having at least one variance in its kinase domain. The administered therapeutic agent may be an antisense oligonucleotides, particularly synthetic oligonucleotides; having chemical modifications from native nucleic acids, or nucleic acid constructs that express such anti-sense molecules as RNA. The antisense sequence is complementary to the mRNA of the targeted EGFR genes, and inhibits expression of the targeted gene products (see e.g. Nyce et al. (1997) Nature 385:720). Antisense molecules inhibit gene expression by reducing the am.ount ofmRNA available for translation, through activation of RNAse H or steric hindrance. One or a combination of antisense molecules may be administered, where a combination may comprise multiple different sequences from a single targeted gene, or sequences that complement several different genes.

A preferred target gene is an EGFR with at least one nucleic acid variance in its kinase domain. Generally, the antisense sequence will have the same species of origin as the animal host.

Antisense molecules may be produced by expression of all or a part of the target gene sequence in an appropriate vector, where the vector is introduced and expressed in the targeted cells. The transcriptional initiation will be oriented such that the antisense strand is produced as an RNA molecule. The anti-sense RNA hybridizes with the endogenous sense strand mRNA, thereby blocking expression of the targeted gene. The native transcriptional initiation region, or an exogenous transcriptional initiation region may be employed.

The promoter may be introduced by recombinant methods in vitro, or as the result of homologous integration of the sequence into a chromosome. Many strong promoters that are active in muscle cells are known in the art, including the β-actin promoter, SV40 early and late promoters, human cytomegalovirus promoter, retroviral LTRs, etc. Transcription vectors generally have convenient restriction sites located near the promoter sequence to provide for the insertion of nucleic acid sequences. Transcription cassettes maybe prepared comprising a transcription initiation region, the target gene or fragment thereof, and a transcriptional termination region. The transcription cassettes may be introduced into a variety of vectors, e.g. plasmid; retrovirus, e.g. lentivirus; adenovirus; and the like, where the vectors are able to transiently or stably be maintained in cells, usually for a period of at least about one day, more usually for a period of at least about several days.

Aptamers are also useful. Aptamers are a promising new class of therapeutic oligonucleotides or peptides and are selected in vitro to specifically bind to a given target with high affinity, such as for example ligand receptors. Their binding characteristics are likely a reflection of the ability of oligonucleotides to form three dimensional structures held together by intramolecular nucleobase pairing. Aptamers are synthetic DNA, RNA or peptide sequences which may be normal and modified (e.g. peptide nucleic acid (PNA), thiophophorylated DNA, etc) that interact with a target protein, ligand (1Ipid, carbohydrate, metabolite, etc). In a further embodiment, RNA aptamers specific for a variant EGFR can be introduced into or expressed in a cell as a therapeutic.

Peptide nucleic acids (PNAs) are compounds that in certain respects are similar to oligonucleotides and their analogs and thus may mimic DNA and RNA. In PNA, the deoxyribose backbone of oligonucleotides has been replaced by a pseudo-peptide backbone (Nielsen et al. 1991 Science 254, 1457-1500). Each subunit, or monomer, has a naturally occurring or non-naturally occurring nucleobase attached to this backbone. One such backbone is constructed of repeating units of N(2-aminoethyl) glycine linked through amide bonds. PNA hybridises with complementary nucleic acids through Watson and Crick base pairing and helix fold. The Pseudo-peptide backbone provides superior hybridization properties (Egholm et al. Nature (1993) 365, 566-568), resistance to enzymatic degradation (Demidov et al. Biochem. Pharmacol. (1994) 48, 1310-1313) and access to a variety of chemical modifications (Nielsen and Haaima Chemical Society Reviews (1997) 73-78). PNAs specific for a variant EGFR can be introduced into or expressed in a cell as a therapeutic. PNAs have been described, for example, in U.S. Application No. 20040063906.

In a preferred embodiment, the EGFR tyrosine kinase inhibitor is erlotinib or gefitinib.

In a first step of the first method according to the invention, the method comprises determining in a sample isolated from said patient the expression levels of the AEG-1 gene.

The term "sample" as used herein, relates to any sample which can be obtained from the subject. Samples may be collected from a variety of sources from a mammal (e.g., a human), including a body fluid sample, or a tissue sample. Samples collected can be human normal and tumor samples, hair, blood, other biofluids, cells, tissues, organs or bodily fluids for example, but not limited to, brain tissue, blood, serum, sputum including saliva, plasma, nipple aspirants, synovial fluids, cerebrospinal fluids, sweat, urine, fecal matter, pancreatic fluid, trabecular fluid, cerebrospinal fluid, tears, bronchial lavage, swabbings, bronchial aspirants, semen, prostatic fluid, precervicular fluid, vaginal fluids, pre-ejaculate, etc. Suitable tissue samples include various types of tumor or cancer tissue, or organ tissue, such as those taken at biopsy.

In a particular embodiment, said sample is any sample containing tumor cells, preferably a tumour tissue sample or a portion thereof or any. Preferably, said tumor tissue sample is a pulmonary tumor tissue sample from a subject suffering from NSCLC who is receiving or has previously received anti-cancer treatmen. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The term "level of expression" or its grammatical equivalent as used herein, means a measurement of the amount of nucleic acid, e.g. RNA or mRNA, or protein of a gene in a subject, or alternatively, the level of activity of a gene or protein in said subject.

As the person skilled in the art understands, the expression levels of AEG-1 gene can be measured by determining the mRNA expression levels of said genes or by determining the protein levels encoded by said genes, i.e. the AEG-1 protein.

Thus, in a particular embodiment, the expression levels of AEG-1 gene are measured by determining mRNA expression levels of said gene.

In order to measure the mRNA levels of the AEG-1 gene, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art [Sambrook, Fischer and Maniatis, Molecular Cloning, a laboratory manual, (2nd ed.), Cold Spring Harbor Laboratory Press, New York, (1989)]. Preferably, care is taken to avoid degradation of the RNA during the extraction process.

The expression level can be determined using mRNA obtained from a formalin-fixed, paraffin-embedded tissue sample coming from a subject as defined above. In this case, the tissue sample is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (Real Time-PCR, SAGE, or TaqMan®) are suitable for use in performing the foregoing aspects of the invention, the mRNA expression levels are often determined by reverse transcription polymerase chain reaction (RT-PCR). The detection can be carried out in individual samples or in tissue microarrays.

In a particular embodiment, the mRNA expression levels of the BRCA1 gene are determined by quantitative PCR, preferably, Real-Time PCR.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA are mRNA derived from housekeeping genes and which code for proteins which are constituvely expressed and carry out essential cellular functions. Examples of housekeeping genes for use in the present invention include β-2-microglobulin, ubiquitin, 18-S ribosomal protein, cyclophilin, GAPDH and β-actin. In a particular embodiment, the control RNA is β-actin mRNA. In one embodiment, relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene

Due to inter-subject variability (e.g. aspects relating to age, race, etc.) it is very difficult (if not practically impossible) to establish absolute reference values for BRCA1 gene. Thus, in this case, the reference values for "high" or "low" expression of the AEG-1 gene are determined by calculating percentiles by conventional means involving the testing of a group of samples isolated from normal subjects (i.e. people with no diagnosis of NSCLC) for the expression levels of the Veg-1. For example, the "high" levels can then be assigned, preferably, to samples wherein expression levels for the AEG-1 gene are equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population. In another embodiment, the expression levels are assigned as "high" or "low" according to their values with respect to the median, wherein the median is the value which separates the higher half of a sample from the lower half. By using the median as cut off value for selecting those patients with high and low expression levels, at most half the population have values less than the median and at most half have values greater than the median.

In another particular embodiment, the expression levels of AEG-1 gene are measured by determining the protein levels encoded by said genes, i.e. the AEG-1 protein.

Practically any conventional method can be used within the context of the present invention to quantify the levels of AEG-1 protein. By way of non-limiting example, the levels of said proteins can be quantified by means of conventional methods, for example, using antibodies with a capacity to specifically bind to AEG-1 protein (or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' y F(ab')2, ScFv, diabodies, triabodies, tetrabodies and humanised antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the BRCA1 protein include techniques of affinity chromatography, binding-ligand assays, etc.

On the other hand, the determination of the level of AEG-1 protein can be carried out by constructing a tissue microarray (TMA) containing the subject samples assembled, and determining the levels of the BRCA1 protein by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cut-offs were selected in order to facilitate reproducibility, and when possible, to translate biological events.

In a second step, the first method of the invention involves the comparing the expression levels of AEG-1 obtained in the first step with a reference sample.

The term "reference sample", "control sample" or their grammatical equivalents, as used herein, relate to a sample, which contains reference nucleic acids or proteins to be used as a source of reference nucleic acids or proteins for the methods of the invention. In a preferred embodiment, the reference sample is obtained by pooling equal amounts of tumor tissue biopsy samples from lung cancer subjects, preferably NSCLC subjects, obtained previous to the adjuvant chemotherapeutic treatment. The AEG-1 nucleic acid or protein levels are then determined in said reference sample and the value obtained is then compared with the levels of the protein or nucleic acid in the test sample. This allows the assignation of the test sample as "low," "normal" or "high" expression. The collection of samples from which the reference level is derived will preferably be constituted from subjects suffering from the same type of cancer, i.e. NSCLC.

The expression "decreased expression", as used herein, refers to a change of expression levels of a given gene with respect to the expression levels in the reference sample of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140% by at least 150%, or more.

The expression "increased expression", as used herein, refers to a change of expression levels of a given gene with respect to the expression levels in the reference sample of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140% by at least 150%, or more.

The expression "positive response" when referred to the treatment with an antitumor agent, as used herein, refers to any response which is substantially better than that obtained with a saline control or placebo. The response can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition of metastasis; (6) enhancement of anti-tumor immune response, possibly resulting in regression or rejection of the tumor; (7) relief, to some extent, of one or more symptoms associated with the tumor; (8) increase in the length of survival following treatment; and/or (9) decreased mortality at a given point of time following treatment.

Positive clinical response may also be expressed in terms of various measures of clinical outcome. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

As used herein the term "a negative response" when referred to the treatment with an EGFR tyrosine kinase inhibitor means that the treatment provides no reduction of the assessed symptoms of the cancer or causes an increase in the symptoms of the cancer being treated.

The term "AEG-1" gene, as used herein, refers to a polynucleotide encoding the astrocyte-elevated gene 1, also known as metadherin, MTDH or Lysine-rich CEACAM-1-associated protein (Lyric). The polynucleotide was originally identified as an oncogene induced in primary human fetal astrocytes infected with human immunodeficiency virus type 1(HIV-1) or treated with HIV envelope glycoprotein (gp120) or tumor necrosis factor-α(TNF-α). The expression of AEG-1 is known to be associated with several signaling pathways including Ha-ras, PI3K/Akt, NF-κB and Wnt/β-catenin. AEG-1 polynucleotides, the levels of which can be used according to the present invention include the human AEG-1 (accession number NM_178812 in the NCBI database), the mouse AEG-1 (accession number NM_026002 in the NCBI database) and the rat AEG-1 (accession number NM_133398 in the NCBI database).

In a preferred embodiment, the method for predicting the response to an EGFR inhibitor is carried out by comparing the expression levels of the AEG-1 gene and of the BRCA1 gene. As shown in example 2 of the present invention, patients with high levels of both genes were considered high-risk whereas patients with low levels of both genes were low-risk and patients with high levels of one and low levels of the other gene were intermediate-risk. Thus, this allows providing a method for predicting the response of a patient suffering lung cancer to an EGFR inhibitor which comprises
(i) determining in a sample isolated from said patient the expression levels of the AEG-1 and BRCA1 genes and
(ii) comparing the expression levels of said AEG-1 and or BRCA1 genes obtained in step (i) with a reference sample
   wherein a decreased expression BRCA1 with respect to a reference sample is indicative of a good response to antitumor treatment or
   wherein an increased expression level of BRCA1 with respect to a reference sample is indicative of a bad response to the antitumor treatment.

The term "BRCA1" or "Breast cancer susceptibility gene 1", as used herein, refers to a tumor suppressor gene identified on the basis of its genetic linkage to familial breast cancers. It encodes a 220-kilodalton nuclear phosphoprotein in normal cells. Mutations of the BRCA1 gene in humans are associated with predisposition to breast and ovarian cancers. In fact, BRCA1 and BRCA2 mutations are responsible for the majority of familial breast cancer. Inherited mutations in the BRCA1 and BRCA2 genes account for approximately 7-10% of all breast and ovarian cancers. Women with BRCA mutations have a lifetime risk of breast cancer between 56-87%, and a lifetime risk of ovarian cancer between 27-44%. In addition, mutations in BRCA1 gene have also been linked to various other tumors including, e. g. , proliferative breast disease (PBD), papillary serous carcinoma of the peritoneum (PSCP), and prostate cancer. Schorge, et al., J.Nat. Ccer/., 90: 841-845 (1998) ; Arason, Am. J. Hum. Genet., 52: 711-717 (1993); Langston, et al., New E7Zg. J ; Med., 334: 137-142 (1996).

In a preferred embodiment, the patients for which a response prediction is carried out according to the method of the invention are patients which show at least a mutation in the EGFR gene. The expression "patients showing at least a mutation in the EGFR gene", as used herein, may be used to refer to patients wherein the tumor contains at least 1 percent, particularly at least 2 percent, 3 percent, 4 percent or 5 percent, particularly at least 10 percent cells which overexpress EGFR (detected e.g. by an immunohistochernistry test such as, for example, the FDA approved EGFR pharmaDx kit ("DAKO" test; DAKO Notrth America, Inc), the Zymed EGFR kit or the Ventana EGFR 3C6 antibody) or which overexpress an EGFR mutant showing altered tyrosine kinase activity.

The terms "ErbB1", "epidermal growth factor receptor" and "EGFR" are used interchangeably herein and refer to a tyrosine kinase which regulate signaling pathways and growth and survival of cells and which shows affinity for the EGF molecule. The ErbB family of receptors consists of four closely related subtypes: ErbB1 (epidermal growth factor receptor [EGFR]), ErbB2 (HER2/neu), ErbB3 (HER3), and ErbB4 (HER4) and variants thereof (e.g. a deletion mutant EGFR as in Humphrey et al. (Proc. Natl. Acad. Sci. USA, 1990, 87:4207-4211).

The EGFR mutations are typically located in the tyrosine kinase domain of the EGF receptor and include mutations conferring sensitivity to tyrosine kinase inhibitors and mutation conferring resistance to EGFR tyrosine kinase inhibitors.

"Mutations conferring sensitivity to EGFR tyrosine kinase inhibitors", as used herein, refer to mutants in the tyrosine kinase domain of EGFR which result in an increased inhibition of the tyrosine kinase activity of EGFR in response to the treatment with inhibitor such as erlotinib. EGFR mutants showing an increased sensitivity to tyrosine kinase inhibitors include, without limitation, mutations at positions L858 in exon 21 such as L858R, L858P, L861Q, or L861 point mutations in the activation loop (exon 21), in-frame deletion/insertion mutations in the ELREA sequence (exon 19) such as the E746-R748 deletion, the E746-A750 deletion, the E746-R748 deletion together with E749Q and A750P substitutions, del L747-E749 deletion combined with the A750P substitution, the L747S substitution in combination with the R748-P753 deletion, the L747-S752 deletion in combination with the E746V substitution, the L747-T751 deletion combined with an serine insertion, the AI insertion at positions M766-A767, the SVA insertion at positions S768-V769, or substitutions in at position 719 in the nucleotide binding loop (exon 18) such as G719A, G719C, G710S.

"Mutations conferring resistance to EGFR tyrosine kinase inhibitors", as used herein, refer to mutants in the tyrosine kinase domain of EGFR which result in a loss of sensitivity of the EGFR tyrosine kinase activity to tyrosine kinase inhibitors both in the wild-type EGFR as well as in EGFR mutants previously showing an increased sensitivity. Mutant EGFR resistant to known EGFR tyrosine kinase inhibitors includes anyone or more EGFR polypeptides, or a nucleotide encoding the same, with a non-wild type residue at one or more positions analogous to c-abl (BCR-ABL) residues that confirm an imatinib resistant phenotype. The residues that when mutated in EGFR confer drug resistance include especially those residues from the kinase domain, including but not limited to, e.g., the P-loop and the activation loop, wherein the mutated residues in the EGFR polypeptide are analogous to c-able residues. Contemplated resistant EGFR mutants have non-wild type residues at the amino acids positions that correspond to residues Lys 714, Leu 718, Ser 720, Ala 722, Phe 723, Thr 725, Ala 750, Thr 790, Leu 792, Met 825, Glu 829, Leu 833, His 870, Thr 892, Phe 961, respectively, in EGFR. Preferred mutations include the T790M point mutation in exon 20 as well as certain insertions in exon 20 such as an NPG Insertion at positions D770-N771, a V insertion at positions P772-H773.

Methods for determining whether a given mutant confers sensitivity or resistance to a tyrosine kinase activity have been described in detail in the prior art and include, among others, a method as described in WO2006091889 based on the detection of the autophosphorylation capacity of EGFR as measured in cells over-expressing EGFR in response to the treatment with a gefintib (Iressa^{™}) or panitumumab.

In a preferred embodiment, the patient shows at least a mutation conferring sensitivity to tyrosine kinase inhibitors and at least one mutation conferring resistance to such inhibitors. In a still more preferred embodiment, the patient shows a first mutation selected from the group of the L858R substitution and the ELREA deletion and a second mutation which is the T790M point mutation in exon 20. In a still yet more preferred embodiment, the patients shows a L858R/T790M mutation.

The mutations and polymorphisms in the EGFR gene are determined using any method known in the art. Typically, the presence of the polymorphism or mutarion is determined in a subject with respect to both copies of the polymorphic site present in the genome. For example, the complete genotype may be characterized as -/-, as -/+, or as +/+, where a minus sign indicates the presence of the reference sequence at the polymorphic site, and the plus sign indicates the presence of a polymorphic variant other than the reference sequence. Any of the detection means described herein may be used to determine the genotype of a subject with respect to one or both copies of the polymorphism present in the subject's genome.

Examples of techniques for detecting differences of at least one nucleotide between two nucleic acids include, but are not limited to, selective oligonucleotide hybridization, selective amplification, or selective primer extension.

For example, oligonucleotide probes may be prepared in which the known polymorphic nucleotide is located centrally (allele-specific probes) and then hybridized to target DNA under conditions which permit hybridization only if a perfect match is found (Saiki et al. (1986) Nature 324:163); Saiki et al (1989) Proc. Natl. Acad. Sci. USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific polymorphic variants are attached to a hybridizing membrane and this membrane is then hybridized with labeled sample nucleic acid. Analysis of the hybridization signal will then reveal the identity of the polymorphic variants of the sample nucleic acid. Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (Human Mutation, 1996, 7:244) and in Kozal et al.(Nature Medicine, 1996, 2:753). The solid phase support is then contacted with a test nucleic acid and hybridization to the specific probes is detected. Accordingly, the identity of numerous polymorphic variants of one or more genes can be identified in a simple hybridization experiment. For example, the identity of the polymorphic variant at any of the polymorphic sites described herein can be determined in a single hybridization experiment.

Alternatively, allele specific amplification technology which depends on selective PCR amplification may be used. For this purpose, it is necessary to have oligonucleotides suitable as primers for the amplification of the fragment of interest. As used herein, the term "primer" refers to a SNP which acts as a point of initiation of template-directed DNA synthesis under appropriate conditions (e.g., in the presence of four different nucleoside triphosphates and a polymerization agent, such as DNA polymerase, RNA polymerase or reverse transcriptase) in an appropriate buffer and at a suitable temperature. The appropriate length of a primer depends on the intended use of the primer, but typically ranges from 15 to 30 nucleotides. Short primer molecules generally require cooler temperatures to form sufficiently stable hybrid complexes with the template. A primer need not be perfectly complementary to the exact sequence of the template, but should be sufficiently complementary to hybridize with it. The term "primer site" refers to the sequence of the target DNA to which a primer hybridizes. The term "primer pair" refers to a set of primers including a 5' (upstream) primer that hybridizes with the 5' end of the DNA sequence to be amplified and a 3' (downstream) primer that hybridizes with the complement of the 3' end of the sequence to be amplified.

Oligonucleotides used as primers for specific amplification may carry the polymorphic variant of interest in the center of the molecule (so that amplification depends on differential hybridization) (Gibbs et al (1989) Nucleic Acids Res. 17:2437-2448) or at the extreme 3' end of one primer where, under appropriate conditions, a mismatch can prevent or reduce polymerase extension (Prossner (1993) Tibtech 11:238; Newton et al. (1989) Nucl. Acids Res. 17:2503). This technique is also termed "PROBE" for Probe Oligo Base Extension. In addition, it may be desirable to introduce a novel restriction site in the region of the mutation to create cleavage-based detection (Gasparini et al., 1992, Mol. Cell Probes 6: 1).

Various detection methods described herein involve first amplifying at least a portion of a gene prior to identifying the polymorphic variant. Amplification can be performed, e.g., by PCR and/or LCR, according to methods known in the art.

Additional amplification methods include, for example, self sustained sequence replication (Guatelli, J. C. et al., 1990, Proc. Natl. Acad. Sci. U.S.A. 87: 1874-1878), transcriptional amplification system (Kwoh, D. Y. et al., 1989, Proc. Natl. Acad. Sci. U.S.A. 86:1 173-1177), Q-Beta Replicase (Lizardi, P. M. et al., 1988, Bio/Technology 6:1 197), or any other nucleic acid amplification method, followed by the detection of the amplified molecules using techniques well known to those of skill in the art. These detection schemes are especially useful for the detection of nucleic acid molecules that may be present in very low numbers.

Any of a variety of sequencing reactions known in the art can be used to directly sequence at least a portion of a gene and detect polymorphic variants by comparing the sequence of the sample sequence with the corresponding control sequence. Exemplary sequencing reactions include those based on techniques developed by Maxam and Gilbert (Proc. Natl. Acad Sci USA, 1977, 74:560) or Sanger (Sanger et al., 1977, Proc. Nat. Acad. Sci. USA, 74:5463). It is also contemplated that any of a variety of automated sequencing procedures may be utilized to identify polymorphic variants (Biotechniques (1995) 19:448), including sequencing by mass spectrometry. See, for example, U.S. Pat. No. 5,547,835 and international patent publication number WO 94/16101, U.S. Pat. No. 5,547,835 and international patent application number WO 94121822 and U.S. Pat. No. 5,605,798 and International Patent Application No. PCT/US96/03651; Cohen et al. (1996) Adv. Chromatogr. 36: 127-162; and Griffin et al. (1993) Appl Biochem Biotechnol 38:147-159. It will be evident to one skilled in the art that, for certain embodiments, the occurrence of only one, two or three of the nucleic acid bases need be determined in the sequencing reaction. For instance, for a single nucleotide run, such as an A-track, only one nucleotide needs to be detected and therefore modified sequencing reactions can be carried out.

Yet other suitable sequencing methods are disclosed, for example, in U.S. Pat. No. 5,580,732 and U.S. Pat. No. 5,571,676.

In some cases, the presence of a specific polymorphic variant in a DNA sample from a subject can be shown by restriction enzyme analysis. For example, a specific polymorphic variant can result in a nucleotide sequence comprising a restriction site which is absent from a nucleotide sequence of another polymorphic variant.

In other embodiments, alterations in electrophoretic mobility may be used to identify the polymorphic variant. For example, single strand conformation polymorphism (SSCP) may be used to detect differences in electrophoretic mobility between polymorphic variants (Orita et al. (1989) Proc Natl. Acad. Sci USA 86:2766, see also Cotton (1993) Mutat Res 285:125-144; and Hayashi (1992) Genet Anal Tech Appl 9:73- 79). Single-stranded DNA fragments of samples and control nucleic acids are denatured and allowed to renature. The secondary structure of single-stranded nucleic acids varies according to sequence and the resulting alteration in electrophoretic mobility enables the detection of even a single base change. The DNA fragments may be labeled or detected with labeled probes. The sensitivity of the assay may be enhanced using RNA (rather than DNA), in which the secondary structure is more sensitive to a change in sequence.

The identity of a polymorphic variant may also be obtained by analyzing the movement of a nucleic acid comprising the polymorphic variant in polyacrylamide gels containing a gradient of denaturant, e.g., denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high- melting GC-rich DNA by PCR. In another embodiment, identification of the polymorphic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g., biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using a biotin ligand, such as avidin. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA which is then detected using OLA.

Methods for detecting mutations in the tyrosine kinase domain of the EGF receptor are known in the art, several corresponding diagnostic tools are approved by the FDA and commercially available, e.g. an assay for the detection of epidermal growth factor receptor mutations in patients with non-small cell lung cancer (Genzyme Corp.; see also Journal of Clinical Oncology, 2006 ASCO Annual Meeting Proceedings (Post-Meeting Edition). VoI 24, No 18S (June 20 Supplement), 2006: Abstract 10060). In a preferred embodiment, the mutations in EGFR are determined in serum samples as described in WO07039705 based on the use of specific Scorpion probes in combination with the Amplification Refractory Mutation System (ARMS) (Nucleic Acids Res., 1989, 17:2503-2516 and Nature Biotechnology, 1999, 17:804-807) or a method as described in WO08009740 based on the selective amplification of the mutant alleles achieved by the use of PNA probes specific for the wild-type variant.

Alternatively, it is also possible to detect the presence or absence of the at least one kinase activity increasing nucleic acid variance involves determining the activation state of downstream targets of EGFR such as Akt and STAT5 as described in WO2005094357.

In one embodiment of the present application, the presence of EGFR mutations can be determined using immunological techniques well known in the art, e.g., antibody techniques such as immunohistochemistry, immunocytochemistry, F ACS scanning, immunoblotting, radioimmunoassays, western blotting, immunoprecipitation, enzyme-linked immunosorbant assays (ELISA), and derivative techniques that make use of antibodies directed against activated downstream targets of EGFR. Examples of such targets include, for example, phosphorylated STAT3, phosphorylated STAT5, and phosphorylated Akt. Using phospho-specific antibodies, the activation status of STAT3, STAT5, and Akt can be determined. Activation of STAT3, STAT5, and Akt are useful as a diagnostic indicator of activating EGFR mutations.

The first method of the invention is suitable for predicting the response of lung cancer patient to an antitumor agent both when the antitumor agent is used as first line treatment in patients which have not been previously treated with chemotherapy as well as when the antitumor agent is used as second line in patients which have been previously been treated with a different treatment but which did not respond or ceased to respond.

The term "first-line treatment" or "first-line therapy" as used herein is an art recognized term and is understood to refer to the first chemotherapy treatment of cancer, which may be combined with surgery and/or radiation therapy, also called primary treatment or primary therapy. Typical antitumor compounds that can be used as forst line for the treatment of lung cancer include, but are not limited to, plant alkaloids, such as vincristine, vinblastine and etoposide; anthracycline antibiotics including doxorubicin, epirubicin, daunorubicin; fluorouracil; antibiotics including bleomycin, mitomycin, plicamycin, dactinomycin; topoisomerase inhibitors, such as camptothecin and its analogues; and platinum compounds, including cisplatin and its analogues, such as carboplatin. Other traditional chemotherapeutic agents suitable for use are known to those of skill in the art and include, asparaginase, busuffan, chlorambucil, cyclophosphamide, cytarabine, dacarbazine, estramustine phosphate sodium, floxuridine, fluorouracil (5-FU), hydroxyurea (hydroxycarbamide), ifosfamide, lornustine (CCNU), mechlorethamine HCl (nitrogen mustard), melphalan, mercaptopurine, methotrexate (MTX), mitomycin, mitotane, mitsxantrone" procarbazine, streptozocin" thioguanine, thiotepa, amsacrine (m-AMSA), azacitidine" hexamethylmeiamine (HMM)" mitoguazone (methyl-GAG; methyl giyoxal bisguanyihydrazone; MGBG), semustine (methyi-CCNU), teniposide (VM-26) and vindesine sulfate.

The term "second-line treatment" or "second-line therapy" as used herein is an art recognized term and is understood to refer to a chemotherapy treatment that is given when initial or primary treatment (first-line or primary therapy) doesn't work, or stops working.

### Therapeutic methods of the invention

The authors of the present invention have observed that, surprisingly, the response of a lung cancer patient to the treatment with an antitumor agent is improved when the patient shows decreased levels of AEG-1 in a sample isolated from said patient. Thus, this result allows a more efficacious treatment of lung cancer patients using when the patients show low AEG-1 levels or BARD1. Accordingly, in another aspect, the invention relates to a method for the treatment of lung cancer in a patient in need thereof which comprises the administration to said patient of an antitumor agent wherein the patient shows reduced AEG-1 levels. Alternatively, the invention provides an antitumor agent for use in the treatment of lung cancer in patients showing reduced AEG-1 levels. Alternatively, the invention provides the use of an an antitumor agent for the manufacture of a medicament for the treatment of lung cancer in a patient which carries at least a mutation in the EGFR receptor and which shows reduced levels of AEG-1.

The term "treating" or its grammatical equivalents as used herein, means achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

The term "lung cancer" has been described above in the context of the first method of the invention. In a preferred embodiment, the lung cancer is non-small cell lung cancer. In a still more preferred embodiment, the non-small cell lung cancer is advanced non-small cell lung cancer.

The term "antitumor agent" has been described in detail above. In a preferred embodiment the antitumor agent is a cisplatin-based composition. In a still more preferred embodiment, the antitumor agent is an EGFR inhibitor.

The term "EGFR inhibitor" has been described in detail in the context of the first method of the invention. In a preferred embodiment, the EGFR is a EGFR tyrosine kinase inhibitor. In a still more preferred embodiment, the EGFR tyrosine kinase inhibitor is erlotinib or gefitinib.

In a preferred embodiment, when the therapeutic method is carried out in patients showing decreased levels of the AEG-1, the levels of BRCA1 are also measured. In this case, the patient to be treated further shows low expression levels of AEG-1 and further shows low expression levels of BRCA1.

In a preferred embodiment, the EGFR tyrosine kinase inhibitor is administered to a patient carrying one or more mutations in the EGFR gene. Mutations in the EGFR gene commonly found in lung tumors are those defined above in the context of the first method of the invention. In a preferred embodiment, the EGFR mutation is selected from the group of a T790M mutation, a L858R mutation, a deletion in exon 19 or a combination thereof. In a preferred embodiment, the patient carries one or more mutations selected from the group of the L858R mutation and a deletion in exon 19, which are known to confer sensibility to tyrosine kinase inhibitors, and the T790M mutation, which confers resistance to tyrosine kinase inhibitors.

The tyrosine kinase inhibitors are then administered to patients showing reduced levels of BRCA1 as known in the art. The route of administration may be intravenous (LV.), intramuscular (LM.), subcutaneous (S.C.), intrademlal (I.D.), intraperitoneal (LP.), intrathecal (LT.), intrapleural, intrauterine, rectal, vaginal, topical, intratumor and the like. The tyrosine kinase inhibitors can be administered parenterally by injection or by gradual infusion over time and can be delivered by peristaltic means.

Administration may be by transmucosal or transdermal means. For transmucosal or transdennal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays, for example, or using suppositories.

For oral administration, the tyrosine kinase inhibitors are formulated into conventional oral administration forms such as capsules, tablets and tonics.

For topical administration, the pharmaceutical composition (inhibitor of kinase activity) is formulated into ointments, salves, gels, or creams, as is generally known in the art.

Typically, the tyrosine kinase inhibitors are administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

The compositions are administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgment of the practitioner and are peculiar to each individual.

The tyrosine kinase inhibitors useful for practicing the methods of the present invention are described herein. Any formulation or drug delivery system containing the active ingredients, which is suitable for the intended use, as are generally known to those of skill in the art, can be used. Suitable pharmaceutically acceptable carriers for oral, rectal, topical or parenteral (including inhaled, subcutaneous, intraperitoneal, intramuscular and intravenous) administration are known to those of skill in the art. The carrier must be pharmaceutically acceptable in the sense of being compatible with the other ingredients of the formulation and not deleterious to the recipient thereof

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects.

Formulations suitable for parenteral administration conveniently include sterile aqueous preparation of the active compound which is preferably isotonic with the blood of the recipient. Thus, such formulations may conveniently contain distilled water, 5% dextrose in distilled water or saline. Useful formulations also include concentrated solutions or solids containing the compound which upon dilution with an appropriate solvent give a solution suitable for parenteral administration above.

For enteral administration, a compound can be incorporated into an inert carrier in discrete units such as capsules, cachets, tablets or lozenges, each containing a predetermined amount of the active compound; as a powder or granules; or a suspension or solution in an aqueous liquid or non-aqueous liquid, e.g., a syrup, an elixir, an emulsion or a draught. Suitable carriers may be starches or sugars and include lubricants, flavorings, binders, and other materials of the same nature.

A tablet may be made by compression or molding, optionally with one or more accessory ingredients. Compressed tablets may be prepared by compressing in a suitable machine the active compound in a free-flowing form, e.g., a powder or granules, optionally mixed with accessory ingredients, e.g., binders, lubricants, inert diluents, surface active or dispersing agents. Molded tablets may be made by molding in a suitable machine, a mixture of the powdered active compound with any suitable carrier.

A syrup or suspension may be made by adding the active compound to a concentrated, aqueous solution of a sugar, e.g., sucrose, to which may also be added any accessory ingredients. Such accessory ingredients may include flavoring, an agent to retard crystallization of the sugar or an agent to increase the solubility of any other ingredient, e.g., as a polyhydric alcohol, for example, glycerol or sorbitol.

Fomulations for rectal administration may be presented as a suppository with a conventional carrier, e.g., cocoa butter or Witepsol S55 (trademark of Dynamite Nobel Chemical, Germany), for a suppository base.

Formulations for oral administration may be presented with an enhancer. Orally-acceptable absorption enhancers include surfactants such as sodium lauryl sulfate, palmitoyl camitine, Laureth-9, phosphatidylcholine, cyclodextrin and derivatives thereof; bile salts such as sodium deoxycholate, sodium taurocholate, sodium glycochlate, and sodium fusidate; chelating agents including EDT A, citric acid and salicylates; and fatty acids (e.g., oleic acid, lauric acid, acylcamitines, mono and diglycerides). Other oral absorption enhancers include benzalkonium chloride, benzethonium chloride, CHAPS (3-(3-cholamidopropyl)-dimethylammonio-lpropanesulfonate), Big-CHAPS (N, N-bis(3-D-gluconamidopropyl)-cholamide), chlorobutanol, octoxynol-9, benzyl alcohol, phenols, cresols, and alkyl alcohols. An especially preferred oral absorption enhancer for the present invention is sodium lauryl sulfate.

Alternatively, the tyrosine kinase inhibitor may be administered in liposomes or microspheres (or microparticles). Methods for preparing liposomes and microspheres for administration to a patient are well known to those of skill in the art. U.S. Pat. No. 4,789,734, the contents of which are hereby incorporated by reference, describes methods for encapsulating biological materials in liposomes. Essentially, the material is dissolved in an aqueous solution, the appropriate phospholipids and lipids added, along with surfactants if required, and the material dialyzed or sonicated, as. necessary. A review of known methods is provided by G. Gregoriadis, Chapter 14,"Liposomes," Drug Carriers in Biology and Medicine, pp. 287-341 (Academic Press, 1979).

Microspheres formed of polymers or proteins are well known to those skilled.in the art, and can be tailored for passage through the gastrointestinal tract directly into the blood stream. Alternatively, the compound can be incorporated and the microspheres, or composite of microspheres, implanted for slow release over a period of time ranging from days to months. See, for example, U.S. Pat. Nos.4,906,474,4,925,673 and 3,625,214, and Jein, TIPS 19:155-157 (1998), the contents of which are hereby incorporated by reference.

In one embodiment, the tyrosine kinase inhibitor can be formulated into a liposome or microparticle which is suitably sized to lodge in capillary beds following intravenous administration. When the liposome or microparticle is lodged in the capillary beds surrounding ischemic tissue, the agents can be administered locally to the site at which they can be most effective. Suitable liposomes for targeting ischemic tissue are generally less than about 200 nanometers and are also typically unilamellar vesicles, as disclosed, for example, in U.S. Pat. No. 5,593,688 to Baldeschweiler, entitled "Liposomal targeting of ischemic tissue," the contents of which are hereby incorporated by reference.

Preferred microparticles are those prepared from biodegradable polymers, such as polyglycolide, polylactide and copolymers thereof. Those of skill in the art can readily determine an appropriate carrier system depending on various factors, including the desired rate of drug release and the desired dosage.

In one embodiment, the formulations are administered via catheter directly to the inside of blood vessels. The administration can occur, for example, through holes in the catheter. In those embodiments wherein the active compounds have a relatively long halflife (on the order of 1 day to a week or more), the formulations can be included in biodegradable polymeric hydrogels, such as those disclosed in U.S. Pat. No. 5,410,016 to Hubbell et al. These polymeric hydrogels can be delivered to the inside of a tissue lumen and the active compounds released over time as the polymer degrades. If desirable, the polymeric hydrogels can have microparticles or liposomes which include the active compound dispersed therein, providing another mechanism for the controlled release of the active compounds.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier and then, if necessary, shaping the product into desired unit dosage form.

The formulations may further include one or more optional accessory ingredient(s) utilized in the art of pharmaceutical formulations, e.g., diluents, buffers, flavoring agents, binders, surface active agents, thickeners, lubricants, suspending agents, preservatives (including antioxidants) and the like.

Compounds of the present methods may be presented for administration to the respiratory tract as a snuff or an aerosol or solution for a nebulizer, or as a micro fine powder for insufflation, alone or in combination with an inert carrier such as lactose. In such a case the particles of active compound suitably have diameters ofless than 50 microns, preferably less than 10 microns, more preferably between 2 and 5 microns.

Generally for nasal administration a mildly acid pH will be preferred. Preferably the compositions of the invention have a pH of from about 3 to 5, more preferably from about 3.5 to about 3.9 and most preferably 3.7. Adjustment of the pH is achieved by addition of an appropriate acid, such as hydrochloric acid.

The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The tyrosine kinase inhibitor to be administered according to the present invention can include pharmaceutically acceptable salts ofthe components therein. Pharmaceutically acceptable salts include the acid addition salts (fonned with the free amino groups of the polypeptide) that are formed with inorganic acids such as, for example, hydrochloric or phosphoric acids, or such organic acids as acetic, tartaric, mandelic and the like. Salts fonned with the free carboxyl groups can also be derived from inorganic bases such as, for example, sodium, potassium, ammonium, calcium or ferric hydroxides, and such organic bases as isopropylamine, trimethylamine, 2- ethyl amino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

If the tyrosine kinase inhibitor is based on RNA interference (e.g, an siRNA), the siRNAs may be chemically synthesized, produced using in vitro transcription, etc. In addition, the siRNA molecule can be customized to individual patients in such a way as to correspond precisely to the mutation identified in their tumor. Since siRNA can discriminate between nucleotide sequences that differ by only a single nucleotide, it is possible to design siRNAs that uniquely target a mutant form of the. EGFR gene that is associated with either a single nucleotide substitution or a small deletion of several nucleotides-both of which have been identified in tumors as described herein.

The delivery of siRNA to tumors can potentially be achieved via any of several gene delivery "vehicles" that are currently available. These include viral vectors, such as adenovirus, lentivirus, herpes simplex virus, vaccinia virus, and retrovirus, as well as chemical-mediated gene delivery systems (for example, liposomes), or mechanical DNA delivery systems (DNA guns). The oligonucleotides to be expressed for such siRNA-mediated inhibition of gene expression would be between 18 and 28 nucleotides in length.

### Kits of the invention

The invention also provides kits which are suitable for the determination of the expression levels of the AEG-1 and BRAD1 genes. These kits are useful for the analyzing a sample from a patient suffering lung cancer and to design personalized therapies for said patients based on the results obtained. Thus, in another aspect, the invention relates to a kit comprising
(i) reagents for detecting the expression levels of AEG-1 and
(ii) reagents for detecting the expression levels of BRCA1.

As used herein, the term "kit" is used in reference to a combination of articles that facilitate a process, method, assay, analysis or manipulation of a sample. These kits provide the materials necessary for carrying out the methods described in the present invention.

The first component of the kit is a set of reagents for detecting the expression levels of AEG-1 gene.

In a particular embodiment, the reagents of the kit are capable of specifically detecting the levels of the mRNA encoded by the AEG-1 gene. In another embodiment, the reagents of the kit are capable of specifically detecting the levels of the AEG-1 protein.

Agents capable of specifically detecting the levels of the mRNA encoded by the AEG-1 gene are:
(i) oligonucleotide primers capable of specifically amplifying a fragment of AEG-1 gene nucleotide sequence; and
(ii) oligonucleotide probes complementary to a fragment of AEG-1 gene gene nucleotide sequence.

As the skilled person understands, the oligonucleotide primers and probes of the kit of the invention can be use in all techniques of gene expression profiling (RT-PCR, SAGE, TaqMan, Real Time- PCR, etc.). The primers and probes forming part of the kit of the invention may be detectably labeled. The kit can also comprise, e.g., a buffering agent, a preservative, or a protein stabilizing agent. The kit can further comprise components necessary for detecting the detectable label (e.g., an enzyme or a substrate). The kit can also contain a control sample or a series of control samples which can be assayed and compared to the test sample. Each component of the kit can be enclosed within an individual container and all of the various containers can be within a single package, along with instructions for interpreting the results of the assays performed using the kit. Agents capable of specifically detecting the expression levels of the AEG-1 protein are antibodies with a capacity to specifically bind to AEG-1 protein (or to fragments thereof containing antigenic determinants). Examples of the antibodies to be employed in the present invention have been previously cited. The antibodies of the kit of the invention can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc. Typically, the kits comprise a second, different antibody which binds to either the polypeptide or the first antibody and is conjugated to a detectable label.

The second component of the kit is a set of reagents for detecting the expression levels of BRCA1 gene. Suitable reagents of the kit suitable for detecting the expression levels of BRCA1 gene include those capable of specifically detecting the levels of the mRNA encoded by the BRCA1 gene. In another embodiment, the reagents of the kit are capable of specifically detecting the levels of the BRCA1 protein. The types of reagents useful for detecting the levels of the mRNA encoded by the BRCA1 gene have been described in detail above in relation to the reagents useful for detecting the levels of the mRNA encoded by the AEG-1 gene. The types of reagents useful for detecting the levels of the BRCA1 protein have been described in detail above in relation to the reagents useful for detecting the levels of the AEG-1 protein.

In a preferred embodiment, the kit further comprises reagents for detecting mutations in the EGFR gene. The reagent may be a probe which is able to distinguish a particular form of the gene or the presence or a particular variance or variances, e.g., by differential binding or hybridization. Thus, exemplary probes include nucleic acid hybridization probes, peptide nucleic acid probes, nucleotide-containing probes which also contain at least one nucleotide analog, and antibodies, e.g., monoclonal antibodies, and other probes as discussed herein. Those skilled in the art are familiar with the preparation of probes with particular specificities. Those skilled in the art will recognize that a variety of variables can be adjusted to optimize the discrimination between two variant forms of a gene, including changes in salt concentration, temperature, pH and addition of various compounds that affect the differential affinity of GC vs. AT base pairs, such as tetramethyl ammonium chloride. (See Current Protocols in Molecular Biology by F. M. Ausubel, R. Brent, R. E. Kingston, D. D. Moore, J. G. Seidman, K Struhl and V. B. Chanda (Editors), John Wiley and Sons.). Such a nucleic acid hybridization probe may span two or more variance sites. Unless otherwise specified, a nucleic acid probe can include one or more nucleic acid analogs, labels or other substituents or moieties so long as the base-pairing function is retained.

The skilled person will appreciate that the nature of the second component of the kit will depend on the method which is used to identify mutations in EGFR gene.

If the detection is to be carried out by differential hybridization, the reagent may be a probe which is able to distinguish a particular form of the gene or the presence or a particular variance or variances, e.g., by differential binding or hybridization. Thus, exemplary probes include nucleic acid hybridization probes, peptide nucleic acid probes, nucleotide-containing probes which also contain at least one nucleotide analog, and antibodies, e.g., monoclonal antibodies, and other probes as discussed herein. Those skilled in the art are familiar with the preparation of probes with particular specificities. Those skilled in the art will recognize that a variety of variables can be adjusted to optimize the discrimination between two variant forms of a gene, including changes in salt concentration, temperature, pH and addition of various compounds that affect the differential affinity of GC vs. AT base pairs, such as tetramethyl ammonium chloride. (See Current Protocols in Molecular Biology by F. M. Ausubel, R. Brent, R. E. Kingston, D. D. Moore, J. G. Seidman, K Struhl and V. B. Chanda (Editors), John Wiley and Sons.). Such a nucleic acid hybridization probe may span two or more variance sites. Unless otherwise specified, a nucleic acid probe can include one or more nucleic acid analogs, labels or other substituents or moieties so long as the base-pairing function is retained.

If the detection involves, prior to the hybridization, an amplification of the target nucleic acid, the kit contains reagents adequate for performing a PCR or, alternatively, ligation chain reaction (LCR) (see, e.g., Landegran, et al., 1988. Science 241: 1077-1080; and Nakazawa, et al., 1994. Proc. Natl. Acad. Sci. USA 91: 360-364), which includes degenerate primers for amplifying the target sequence, the primers corresponding to one or more conserved regions of the gene,

Alternative amplification methods include: self sustained sequence replication (see, Guatelli, et al., 1990. Proc. Natl. Acad. Sci. USA 87: 1874-1878), transcriptional amplification system (see, Kwoh, et al., 1989. Proc. Natl. Acad. Sci. USA 86: 1173-1177); Qb Replicase (see, Lizardi, et al, 1988. BioTechnology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

Primers useful according to the present invention are designed using amino acid sequences of the protein or nucleic acid sequences of the kinase domain of the EGFR gene gene as a guide. The primers are designed in the homologous regions of the gene wherein at least two regions of homology are separated by a divergent region of variable sequence, the sequence being variable either in length or nucleic acid sequence. For example, the identical or highly, homologous, preferably at least 80 percent -85 percent more preferably at least 90-99 percent homologous amino acid sequence of at least about 6, preferably at least 8-10 consecutive amino acids. Most preferably, the amino acid sequence is 100 percent identical. Forward and reverse primers are designed based upon the maintenance of codon degeneracy and the representation of the various amino acids at a given position among the known gene family members. Degree of homology as referred to herein is based upon analysis of an amino acid sequence using a standard sequence comparison software, such as protein-BLAST using the default settings (http://www.ncbi.nlm.nih.gov/BLAST/).

The primers may be labeled using labels known to one skilled in the art. Such labels include, but are not limited to radioactive, fluorescent, dye, and enzymatic labels.

In an alternative embodiment, mutations in a EGFR gene from a sample cell can be identified by alterations in restriction enzyme cleavage patterns in which case the kits of the invention further comprise restriction endonucleases capable of discriminating the wild-type and the mutated EGFR gene.

Other methods for detecting mutations in the EGFR gene include methods in which protection from cleavage agents is used to detect mismatched bases in RNA/RNA or RNA/DNA heteroduplexes. See, e.g., Myers, et al., 1985. Science 230: 1242. In general, the art technique of "mismatch cleavage" starts by providing heteroduplexes of formed by hybridizing (labeled) RNA or DNA containing the wild-type EGFR sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, e.g., Cotton, et al., 1988. Proc. Natl. Acad. Sci. USA 85: 4397; Saleeba, et al., 1992. Methods Enzymol. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in EGFR cDNAs obtained from samples of cells. For example, the mutY enzyme of E. coli cleaves A at G/A mismatches and the thymidine DNA glycosylase from HeLa cells cleaves T at G/T mismatches. See, e.g., Hsu, et al., 1994. Carcinogenesis 15: 1657-1662. According to an exemplary embodiment, a probe based on a mutant EGFR sequence, e.g., a DEL-1 through DEL-5, G719S, G857V, L883S or L858R EGFR sequence, is hybridized to a cDNA or other DNA product from a test cell(s). The duplex is treated with a DNA mismatch repair enzyme, and the cleavage products, if any, can be detected from electrophoresis protocols or the like. See, e.g., U.S. Pat. No. 5,459,039.

In other embodiments, alterations in electrophoretic mobility will be used to identify mutations in EGFR genes. For example, single strand conformation polymorphism (SSCP) may be used to detect differences in electrophoretic mobility between mutant and wild type nucleic acids. See, e.g., Orita, et al., 1989. Proc. Natl. Acad. Sci. USA: 86: 2766; Cotton, 1993. Mutat. Res. 285: 125-144; Hayashi, 1992. Genet. Anal. Tech. Appl. 9: 73-79. Single-stranded DNA fragments of sample and control EGFR nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. See, e.g., Keen, et al., 1991. Trends Genet. 7: 5.

In yet another embodiment, the movement of mutant or wild-type fragments in polyacrylamide gels containing a gradient of denaturant is assayed using denaturing gradient gel electrophoresis (DGGE). See, e.g., Myers, et al., 1985. Nature 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. See, e.g., Rosenbaum and Reissner, 1987. Biophys. Chem. 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. See, e.g., Saiki, et al., 1986. Nature 324: 163; Saiki, et al., 1989. Proc. Natl. Acad. Sci. USA 86: 6230. Such allele specific oligonucleotides are hybridized to PCR amplified target DNA or a number of different mutations when the oligonucleotides are attached to the hybridizing membrane and hybridized with labeled target DNA.

In a preferred embodiment, the kit as of the invention comprises reagents for the identificaciont of an mutation in the EGFR gene selected from the group of a T790M mutation, a L858R mutation, a deletion in exon 19 or a combination thereof.

Another component which can be present in the kit is a packing which allows maintaining the reagents within determined limits. Suitable materials for preparing such packings include glass, plastic (polyethylene, polypropylene, polycarbonate and the like), bottles, vials, paper, sachets and the like. The kit of the invention can additionally contain instructions for using the reagents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

In one embodiment, a kit for the detection the expression levels of BRCA1 gene and of variances in the kinase domain of erbB1 is provided on a solid support is described. The kit can include, e.g. the materials and reagents for detecting a plurality of variances in one assay. The kit can include e.g. a solid support, oligonucleotide primers for a specific set of target polynucleotides, polymerase chain reaction reagents and components, e.g. enzymes for DNA synthesis, labeling materials, and other buffers and reagents for washing. The kit may also include instructions for use of the kit to amplify specific targets on a solid support. Where the kit contains a prepared solid support having a set of primers already fixed on the solid support, e.g. for amplifying a particular set of target polynucleotides, the design and construction of such a prepared solid support is described above. The kit also includes reagents necessary for conducting a PCR on a solid support, for example using an in situ-type or solid phase type PCR procedure where the support is capable of PCR amplification using an in situ-type PCR machine. The PCR reagents, included in the kit, include the usual PCR buffers, a thermostable polymerase (e.g. Taq DNA polymerase), nucleotides (e.g. dNTPs), and other components and labeling molecules (e.g. for direct or indirect labeling as described above). The kits can be assembled to support practice of the PCR amplification method using immobilized primers alone or, alternatively, together with solution phase primers. Alternatively, the kit may include a solid support with affixed oligonucleotides specific to BRCA1 and any number of EGFR variances as defined above. A test biological sample may be applied to the solid support, under selective hybridization conditions, for the determination of the BRCA1 expression levels and the presence or absence of mutations in EGFR.

The solid phase support of the present invention can be of any solid materials and structures suitable for supporting nucleotide hybridization and synthesis. Preferably, the solid phase support comprises at least one substantially rigid surface on which oligonucleotides or oligonucleotide primers can be immobilized. The solid phase support can be made of, for example, glass, synthetic polymer, plastic, hard non-mesh nylon or ceramic. Other suitable solid support materials are known and readily available to those of skill in the art. The size of the solid support can be any of the standard microarray sizes, useful for DNA microarray technology, and the size may be tailored to fit the particular machine being used to conduct a reaction of the invention. Methods and materials for derivatization of solid phase supports for the purpose of immobilizing oligonucleotides are known to those skill in the art and described in, for example, U.S. Pat. No. 5,919,523, the disclosure of which is incorporated herein by reference.

The solid support can be provided in or be part of a fluid containing vessel. For example, the solid support can be placed in a chamber with sides that create a seal along the edge of the solid support so as to contain the polymerase chain reaction (PCR) on the support. In a specific example the chamber can have walls on each side of a rectangular support to ensure that the PCR mixture remains on the support and also to make the entire surface useful for providing the primers.

The oligonucleotide or oligonucleotide primers of the invention are affixed, immobilized, provided, and/or applied to the surface of the solid support using any available means to fix, immobilize, provide and/or apply the oligonucleotides at a particular location on the solid support. For example, photolithography (Affymetrix, Santa Clara, Calif) can be used to apply the oligonucleotide primers at particular position on a chip or solid support, as described in the U.S. Pat. Nos. 5,919,523, 5,837,832, 5,831,070, and 5,770,722, which are incorporated herein by reference. The oligonucleotide primers may also be applied to a solid support as described in Brown and Shalon, U.S. Pat. No. 5,807,522 (1998). Additionally, the primers may be applied to a solid support using a robotic system, such as one manufactured by Genetic MicroSystems (Woburn, Mass.), GeneMachines (San Carlos, Calif.) or Cartesian Technologies (Irvine, Calif.).

In one aspect of the invention, solid phase amplification of target polynucleotides from a biological sample is performed, wherein multiple groups of oligonucleotide primers are immobilized on a solid phase support. In a preferred embodiment, the primers within a group comprises at least a first set of primers that are identical in sequence and are complementary to a defined sequence of the target polynucleotide, capable of hybridizing to the target polynucleotide under appropriate conditions, and suitable as initial primers for nucleic acid synthesis (i.e., chain elongation or extension). Selected primers covering a particular region of the reference sequence are immobilized, as a group, onto a solid support at a discrete location. Preferably, the distance between groups is greater than the resolution of detection means to be used for detecting the amplified products. In a preferred embodiment, the primers are immobilized to form a microarray or chip that can be processed and analyzed via automated, processing. The immobilized primers are used for solid phase amplification of target polynucleotides under conditions suitable for a nucleic acid amplification means. In this manner, the presence or absence of a variety of potential variances in the kinase domain of the erbB1 gene can be determined in one assay.

An in situ-type PCR reactions on the microarrays can be conducted essentially as described in e.g. Embretson et al, Nature 362:359-362 (1993); Gosden et al, BioTechniques 15(1):78-80 (1993); Heniford et al Nuc. Acid Res. 21(14):3159-3166 (1993); Long et al, Histochemistry 99:151-162 (1993); Nuovo et al, PCR Methods and Applications 2(4):305-312 (1993); Patterson et al Science 260:976-979 (1993).

Alternatively, variances in the kinase domain of erbB1 can be determined by solid phase techniques without performing PCR on the support. A plurality of oligonucleotide probes, each containing a distinct variance in the kinase domain of erbB1, in duplicate, triplicate or quadruplicate, may be bound to the solid phase support. The presence or absence of variances in the test biological sample may be detected by selective hybridization techniques, known to those of skill in the art and described above.

### Further aspects of the invention

[1] A method for predicting the response of a patient suffering lung cancer to an antitumor treatment which comprises
   (i) determining in a sample isolated from said patient the expression levels of the AEG-1 gene and
   (ii) comparing the expression levels of said AEG-1 gene obtained in step (i) with a reference sample
      wherein a decreased expression level of the AEG-1 gene with respect to a reference sample is indicative of a good response to the antitumor treatment or wherein an increased expression level of AEG-1 gene with respect to a reference sample is indicative of a bad response to the antitumor treatment.
[2] A method as defined in [1] further comprising determining the expression levels of BRCA1,
   wherein a decreased expression BRCA1 with respect to a reference sample is indicative of a good response to antitumor treatment or
   wherein an increased expression level of BRCA1 with respect to a reference sample is indicative of a bad response to the antitumor treatment.
[3] A method as defined in [1] or [2] wherein the lung cancer is non-small cell lung cancer.
[4] A method as defined in [3] wherein the non-small cell lung cancer is advanced non-small cell lung cancer.
[5] A method as defined in any of [1] to [4] wherein the antitumor treatment is a cisplatin-based chemotherapy.
[6] A method as defined in any of [1] to [4] wherein the antitumor treatment is an EGFR inhibitor
[7] A method as defined in [6] wherein the EGFR inhibitor is a tyrosine kinase inhibitor.
[8] A method as defined in [7] wherein the EGFR tyrosine kinase inhibitor is erlotinib or gefitinib.
[9] A method as defined in any of [6] to [8] wherein the tumor comprises at least one mutation in the EGFR gene.
[10] A method as defined in [9] wherein the at least one mutation in the EGFR gene is selected from the group of a mutation conferring sensitivity to a tyrosine kinase inhibitor and a mutation conferring resistance to a tyrosine kinase inhibitor.
[11] A method as defined in [10] wherein the mutation in the EGFR gene conferring sensitivity to a tyrosine kinase inhibitor is selected from the group of a L858R mutation and an ELREA deletion in exon 19 and/or the mutation conferring resistance to a tyrosine kinase inhibitor is the T790M mutation.
[12] A method as defined in any of [1] to [11] wherein the sample contains tumour cells.
[13] An antitumor composition for use in the treatment of lung cancer wherein the patient to be treated shows low expression levels of the AEG-1 gene.
[14] An antitumor composition for use according to [13] wherein the patient to be treated further shows low expression levels of BRCA1.
[15] An antitumor composition for use according to any of [13] or [14] herein the lung cancer is non-small cell lung cancer.
[16] An antitumor composition for use according to [15] wherein the non-small cell lung cancer is advanced non-small cell lung cancer.
[17] An antitumor composition for use according to any of [13] to [16] wherein the antitumor composition is a cisplatin-based chemotherapy.
[18] An antitumor composition for use according to any of [13] to [16] wherein the antitumor composition is an EGFR inhibitor.
[19] An antitumor composition for use according to [17] wherein the EGFR inhibitor is EGFR tyrosine kinase inhibitor.
[20] An antitumor composition for use according to [18] wherein the EGFR tyrosine kinase inhibitor is erlotinib or gefitinib.
[21] An EGFR tyrosine kinase inhibitor for use according to any of [17] to [19] wherein the patient shows at least one mutation in the EGFR gene.
[22] An EGFR tyrosine kinase inhibitor for use according to [20] wherein the at least one mutation in the EGFR gene is selected from the group of a mutation conferring sensitivity to a tyrosine kinase inhibitor and a mutation conferring resistance to a tyrosine kinase inhibitor.
[23] An EGFR tyrosine kinase inhibitor as defined in [21] wherein the mutation in the EGFR gene conferring sensitivity to a tyrosine kinase inhibitor is selected from the group of a L858R mutation and an ELREA deletion in exon 19 and/or the mutation conferring resistance to a tyrosine kinase inhibitor is the T790M.
[24] A kit comprising
   (i) reagents for detecting the expression levels of AEG-1 and
   (ii) reagents for detecting the expression levels of BRCA1.
[25] A kit as defined in [23] further comprising reagents for detecting the presence of a mutation in the EGFR gene.
[26] A kit as defined in [24] wherein said EGFR mutation is selected from the group of a T790M mutation, a L858R mutation, an ELREA deletion in exon 19 or a combination thereof.

The invention is described in detail by way of the following examples which are to be considered as merely illustrative and not limitative of the scope of the invention.

### EXAMPLE 1

### Identification of genes potentially involved in erlotinib resistance

In order to explore genes potentially involved in erlotinib resistance, the integrated digital technology NanoString, was used to examine expression levels of 48 genes in a single reaction. Several genes involved in homologous recombination repair were included in the 48-gene assessment, including the E3 ubiquitin ligases RNF8, RNF168 and RAP80.

Methods: CodeSets (Reporter and Capture probe sets) for 48 genes were custom-designed by NanoString Technologies. 100ng of total RNA for each sample was mixed with the reagents and hybridized at 65° overnight. Samples were processed and analyzed with the nCounter Prep Station (NanoString Technologies). Data analysis was performed adjusting the expression values according to positive control values and normalizing the data according to housekeeping genes.

### EXAMPLE 2

### Astrocyte elevated gene 1 (AEG-1) mRNA expression in non-small-cell lung cancer (NSCLC) patients (p) with epidermal growth factor receptor (EGFR) mutations

Table 1 shows the characteristics of the 55 NSCLC patients with EGFR mutations were included in the study.

**Table 1. Patient characteristics for 55 erlotinib-treated advanced NSCLC patients with EGFR mutations**

| | N=55 |
|---|---|
| | N (%) |
| Age | 67 (22-85) |
| Sex | |
| Male | 16 (29.1) |
| Female | 39 (70.9) |
| Race | |
| Asian | 1 (1.8) |
| Caucasian | 54 (98.1) |
| Smoking history | |
| Ex-smoker | 12 (21.8) |
| Current smoker | 4 (7.3) |
| Never smoked | 39 (70.9) |
| ECOG PS | |
| 0 | 17 (30.9) |
| 1 | 29 (52.7) |
| ≥2 | 9 (16.4) |
| Histology | |
| Adeno | 45 (81.8) |
| BAC | 7 (12.7) |
| LCC | 3 (5.5) |
| Stage | |
| IIIB | 3 (5.5) |
| IV | 52 (94.5) |
| Metastasis site | |
| Bone | 16 (29.1) |
| Brain | 6 (10.9) |
| Erlotinib treatment line | |
| First | 31 (56.4) |
| Second | 24 (43.6) |
| Type of EGFR mutation | |
| del 19 | 34(61.8) |
| L858R | 21 (38.2) |
| T790M mutation | |
| Present | 22 (40) |
| Absent | 33 (60) |
| Response | |
| CR | 7 (14.6) |
| PR | 25 (52.1) |
| CR+PR | 32 (66.7) |
| SD | 11 (22.9) |
| PD | 5 (10.4) |
| NE | 7 |

The majority of patients were females and never-smokers; 16.4% had PS 2. All the patients had distant metastases, except three patients with only malignant pleural effusion. Bone metastases were present in 29.1% of the patients. Thirty-one patients received erlotinib as first-line treatment, and 24 received erlotinib following progression to chemotherapy. EGFR exon 19 deletion was detected in 61.8% of the cases, and the T790M mutation was present before erlotinib treatment in 40%. Complete response was attained in 14.6% and partial response in 52.1 % of the patients, for an overall response rate of 66.7% (Table 1). Overall median PFS was 16 months (95% CI, 8.1-23.9 months), and median survival was 29 months (95% CI, 22.3-35.7 months).

Terciles of AEG-1 were derived, and patients were subdivided into three groups based on low, intermediate or high levels of gene expression to compare PFS. In the univariate analysis, AEG-1 expression levels were associated with PFS (Table 2).

**Table 2: Progression-free survival by gene expression terciles**

| Gene | N | Median | 95% CI | P |
|---|---|---|---|---|
| AEG1 | | | | **0.01** |
| ≤0.384 | 19 | 22 | 5.9-38.1 | |
| 0.3 84-0.474 | 18 | 23 | 10.6-35.4 | |
| >0.474 | 18 | 6 | 0-14.9 | |

Since patients with low and intermediate (23 months) AEG-1 levels had similar median PFS (22 vs. 23 months) (Table 2), these two groups of patients were combined for further statistical analysis. PFS for patients with low/intermediate AEG-1 expression was 22 months (95% CI, 10.3-33.7 months), compared to 6 months (95% CI, 0-14.9 months) for patients with high AEG-1 expression (P=0.003) (Fig. 1). PFS was longer in patients with low AEG-1 expression (27 vs 12 m; P=0.003). Median survival (MS) was not reached for p with low AEG-1 levels and was 24 m for patients with high levels (P=0.08).

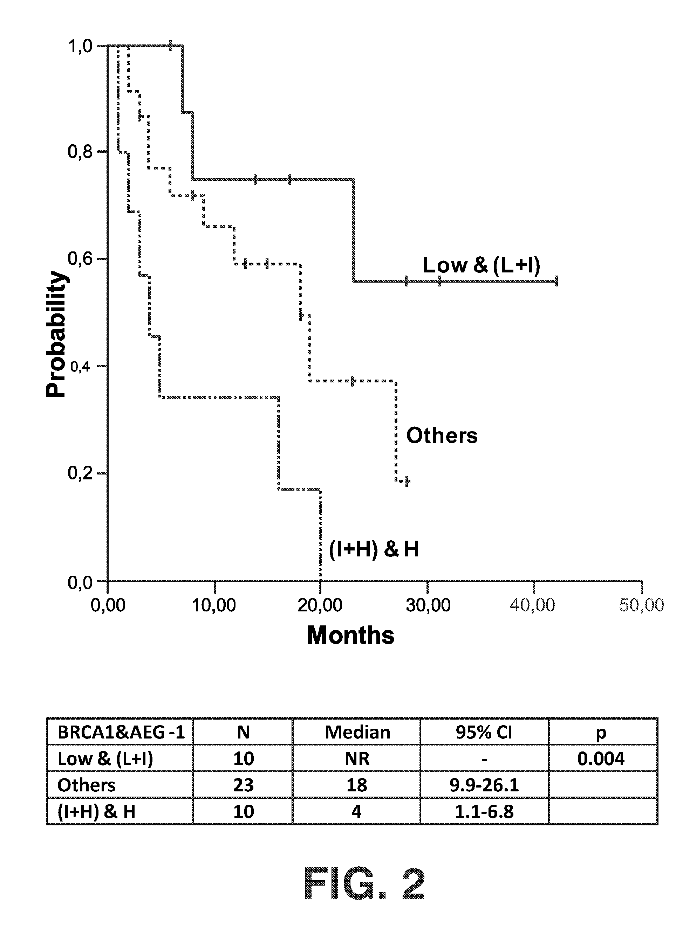

It was further investigated the combined effect of BRCA1 and AEG-1 expression on outcomes in the present cohort of patients. PFS for patients with low BRCA1 and low/intermediate AEG-1 levels was not reached, while it was 4 months (95% CI, 1.1-6.8 months) for those with intermediate/high BRCA1 and high AEG-1 levels, and 18 months (95% CI, 9.9-26.1 months) for those with other combinations (P=0.004) (Fig. 2). Based on these results, a BRCAl/AEG-1 combined risk group model was generated, shown in Table 3, classifying patients into low-, intermediate- and high-risk groups.

**Table 3: Classification of patients into risk groups based on a two-model**

| | | AEG-1 | | |
|---|---|---|---|---|
| | | Low | Intermediate | High |
| BRCA1 | Low | Low Risk | Low Risk | Medium Risk |
| | Intermediate | Medium Risk | Medium Risk | High Risk |
| | High | Mediun Risk | High Risk | High Risk |

Response to erlotinib was significantly different for the three groups (Table 4). Complete response was attained in 42.9% of patients in the low-risk group, compared to 3% in the intermediate-risk and 0% in the high-risk group (P=0.02). Overall response rates were 85.7% in the low-risk, 81 % in the intermediate-risk, and 2% in the high-risk group (P=0.004) (Table 4).

**Table 4: Response rate according to BRCA1/AEG-1 risk group**

| | BRCAl/AEG-1 Low&(I+H) | BRCA1/AEG-1 Others | BRCA1/AEG-1 (I+H)&H | p |
|---|---|---|---|---|
| CR | 3(42.9) | 3(14.3) | 0(0) | 0.02 |
| PR | 3(42.9) | 14(66.7) | 2(22.2) | |
| SD | 1(14.3) | 3(14.3) | 4(44.4) | |
| PD | 0(0) | 1(4.8) | 3(33.3) | |
| CR | 6(85.7) | 17(81) | 2(22.2) | 0.004 |
| PR | 1(14.3) | 4(19) | 7(77.8) | |

### Multivariate analysis

In the multivariate analysis for PFS, only the T790M mutation and the BRCA1/AEG-1 risk group emerged as significant variables (Table 5). The hazard ratio (HR) for the presence of the T790M mutation was 2.35 (95% CI, 1-5.61; P=0.05). The HR for the high-risk group was 7.29 (95% CI, 1.73-30.75; P=0.007) (Table 5).

**Table 5. Multivariate analysis of PFS**

| | HR | 95% CI | p |
|---|---|---|---|
| Brain metastasis | | | |
| No | 1 ref. | | |
| Yes | 2.84 | 0.78-10.36 | 0.14 |
| Bone metastasis | | | |
| No | 1 ref. | | |
| Yes | 1.15 | 0.35-3.79 | 0.81 |
| T790M mutation | | | |
| Absent | 1 ref. | | |
| Present | 2.35 | 1-5.61 | 0.05 |
| BRCA1/AEG-1 risk groups | | | |
| Low-risk | 1 ref. | | |
| Intermediate-risk | 2.02 | 0.52-7.84 | 0.31 |
| High-risk | 7.29 | 1.73-30.75 | 0.007 |

Conclusions: The two-gene risk model based on AEG-1 and BRCA1 mRNA expression was strongly associated with clinical outcome, with significantly shorter PFS and MS in the high-risk group. This model can be useful for the proper individualized management of p with EGFR mutation.

### EXAMPLE 3

### AEG-1 and BRCA1 expression levels in non-small-cell lung cancer (NSCLC) patients treated with chemotherapy.

60 patients suffering advanced NSCLC were analysed.

**Table 6. Patient characteristics for 60 chemotherapy-treated advanced NSCLC patients.**

| | **NUMBER** **(N: 60)** | **PERCENTAGE** **(%)** |
|---|---|---|
| **GENDER** | | |
| Male | 36 | 60 |
| Female | 24 | 40 |
| **AGE (Min-Max)** | 58 years (range 29-76) | |
| HISTOLOGY | | |
| Large cell | 8 | 13.3 |
| Adenocarcinoma | 39 | 65 |
| Squamous cell | 13 | 21.7 |
| **SMOKING STATUS** | | |
| Smokers | 22 | 36.7 |
| Never Smokers | 7 | 11.7 |
| Former Smokers | 20 | 33.3 |
| Unknown | 11 | 18.3 |
| **NUMBER OF CHEMOTHERAPY LINES** | | |
| 0 | 4 | 6.7 |
| 1 | 14 | 23.3 |
| 2 | 21 | 35 |
| 3 | 12 | 20 |
| 4 | 7 | 11.7 |
| 5 | 2 | 3.3 |
| **METASTATIS LOCALIZATION** | | |
| Lung | 25 | 41.7 |
| Bone | 23 | 38.3 |
| Brain | 10 | 10 |
| Liver | 4 | 6.7 |
| Pleural | 8 | 13.3 |
| Suprarrenal | 8 | 13.3 |
| Skin | 1 | 1,7 |
| Others | 4 | 6.7 |
| **1st LINE THERAPY** | | |
| Chemotherapy (CT) | 48 | 80 |
| Erlotinib (TKI) | 7 | 11.7 |
| CUT + SKI | 1 | 1,7 |
| **RESPONSE** | 51 | 85 |
| CR | 5 | 8.3 |
| PR | 28 | 46.7 |
| SD | 5 | 8.3 |
| PD | 13 | 21,7 |
| **EGFR MUTATIONS** | 9/51 | 17,65 |
| Del 19 | 7 | 13.73 |
| L858R | 2 | 3.92 |
| **KRAS MUTATIONS** | 10/56 | 17.86 |
| **MEDIAN MONTHS OF FOLLOW UP** | 17.14 Months | 2 Months to 152.17 Months |

No significant correlations were observed between expression levels of BRCA1 or AEG-1 and the presence of mutations in EGFR.

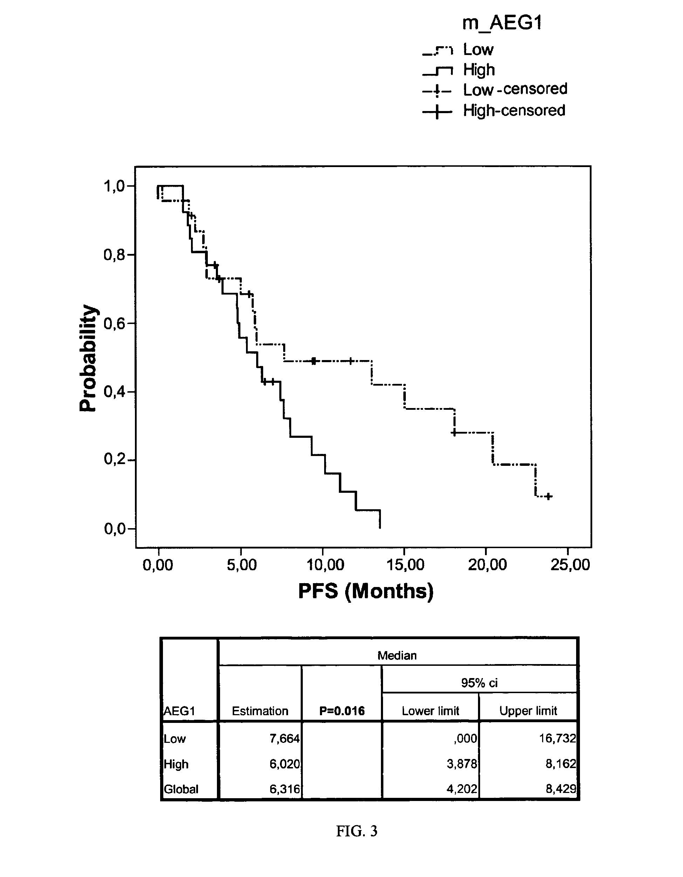

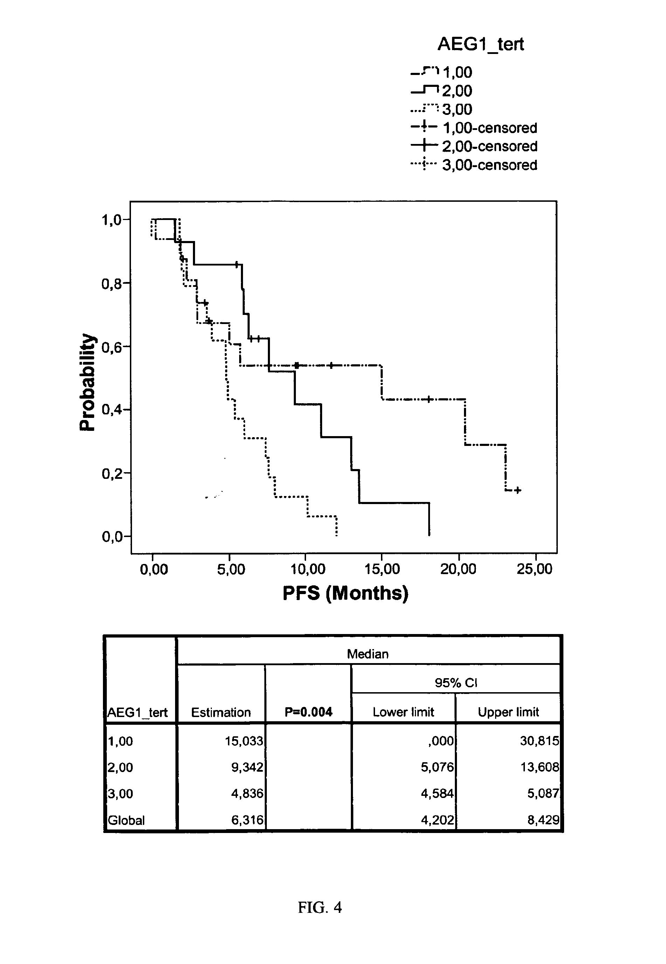

A significant correlation was observed between expression levels of AEG-1 and progression-free survival (see figures 3 and 4). In particular, PFS was of 12 month in patients in the lower tertile of AEG-1 expression and 4 months in patients in the upper tertile of AEG-1 expression.

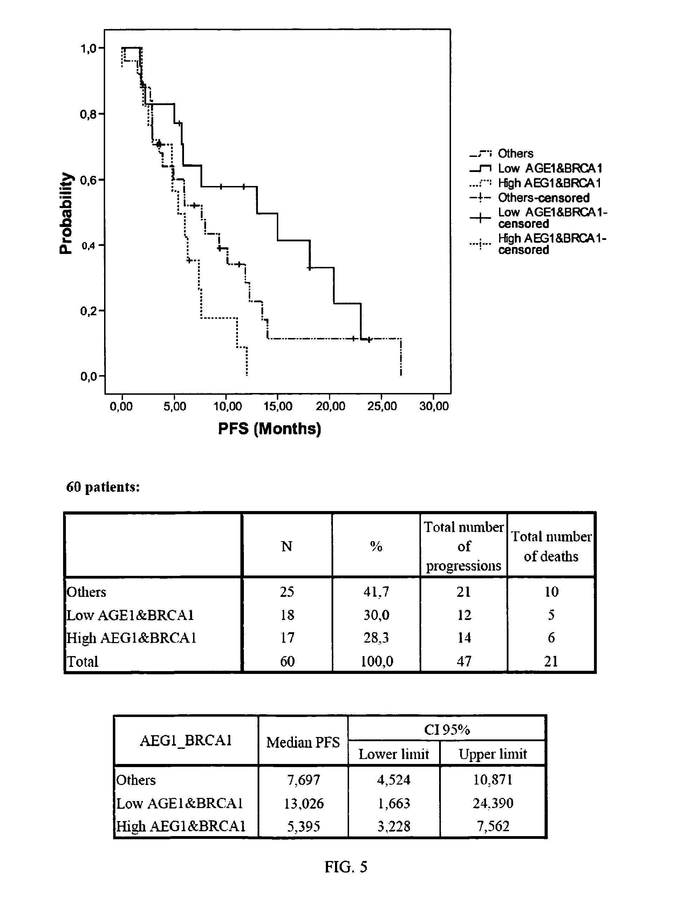

Moreover, a significant correlation was also observed between expression levels of AEG-1 and BRCA1 and progression free survival (see figure 5). In particular, PFS in the group of patients expressing high levels of both genes was of 5,395 months whereas PFS in patients showing decreased levels of AEG-1 and BRCA1 was of 13 months.

## Claims

1. A method for predicting the response of a patient suffering lung cancer to an antitumor composition which comprises
(i) determining in a sample isolated from said patient the expression levels of the AEG-1 gene and
(ii) comparing the expression levels of said AEG-1 gene obtained in step (i) with a reference sample
wherein a decreased expression level of the AEG-1 gene with respect to a reference sample is indicative of a good response to the antitumor treatment or wherein an increased expression level of AEG-1 gene with respect to a reference sample is indicative of a bad response to the antitumor composition.

2. A method as defined in claim 1 further comprising determining the expression levels of BRCA1,
wherein a decreased expression BRCA1 with respect to a reference sample is indicative of a good response to antitumor treatment or
wherein an increased expression level of BRCA1 with respect to a reference sample is indicative of a bad response to the antitumor composition.

3. A method as defined in claims 1 or 2 wherein the lung cancer is non-small cell lung cancer.

4. A method as defined in any of claims 1 to 4 wherein the antitumor composition is a cisplatin-based chemotherapy.

5. A method as defined in any of claims 1 to 4 wherein the antitumor composition is an EGFR inhibitor

6. A method as defined in claim 5 wherein the tumor comprises at least one mutation in the EGFR gene.

7. A method as defined in claim 6 wherein the at least one mutation in the EGFR gene is selected from the group of a mutation conferring sensitivity to a tyrosine kinase inhibitor and a mutation conferring resistance to a tyrosine kinase inhibitor.

8. A method as defined in any of claims 1 to 7 wherein the sample contains tumour cells.

9. An antitumor composition for use in the treatment of lung cancer wherein the patient to be treated shows low expression levels of the AEG-1 gene.

10. An antitumor composition for use according to claim 9 wherein the patient to be treated further shows low expression levels of BRCA1.

11. An antitumor composition for use according to any of claims 9 or 10 herein the lung cancer is non-small cell lung cancer.

12. An antitumor composition for use according to any of claims 8 to 11 wherein the antitumor composition is a cisplatin-based chemotherapy.

13. An antitumor composition for use according to any of claims 8 to 11 wherein the antitumor composition is an EGFR inhibitor.

14. An antitumor composition for use according to claim 13 wherein the patient shows at least one mutation in the EGFR gene.

15. An antitumor composition for use according to claim 14 wherein the at least one mutation in the EGFR gene is selected from the group of a mutation conferring sensitivity to a tyrosine kinase inhibitor and a mutation conferring resistance to a tyrosine kinase inhibitor.

16. A kit comprising
(i) reagents for detecting the expression levels of AEG-1 and
(ii) reagents for detecting the expression levels of BRCA1.

17. A kit as defined in claim 16 further comprising reagents for detecting the presence of a mutation in the EGFR gene.

18. A kit as defined in claim 17 wherein said EGFR mutation is selected from the group of a T790M mutation, a L858R mutation, an ELREA deletion in exon 19 or a combination thereof.
